# EUROPEAN PATENT SPECIFICATION

(11) **EP 3 328 413 B1**
(45) Date of publication and mention of the grant of the patent: **04.09.2024**
(21) Application number: 16831435.9
(22) Date of filing: 29.07.2016
(51) Int. Cl.: A61K 38/17, C07K 14/435, C12N 15/63, A01N 1/02, A61K 35/28

(54) **MATERIALS AND METHODS FOR TREATING AND EVALUATING ISCHEMIC AND/OR REPERFUSION-INJURED TISSUE AND/OR TISSUE SUSCEPTIBLE TO SAME**
MATERIALIEN UND VERFAHREN ZUR BEHANDLUNG UND BEURTEILUNG VON ISCHÄMISCHEM UND/ODER REPERFUSIONSBESCHÄDIGTEM GEWEBE UND/ODER DAFÜR ANFÄLLIGEM GEWEBE
MATÉRIAUX ET MÉTHODES POUR LE TRAITEMENT ET L'ÉVALUATION DE TISSU À LÉSIONS ISCHÉMIQUES ET/OU DE REPERFUSION ET/OU TISSU SENSIBLE À CELLES-CI

(30) Priority: 29.07.2015 US 201562198661 P; 28.04.2016 US 201662328994 P
(43) Date of publication of application: 06.06.2018
(73) Proprietor: Indiana University Research and Technology Corporation, Bloomington, IN 47405 (US)
(72) Inventor: MARCH, Keith L., Carmel, Indiana 46032 (US); WANG, Meijing, Carmel, Indiana 46033 (US)
(74) Representative: HGF
(86) International application number: PCT/US2016/044799
(87) International publication number: WO 2017/019986

(56) References cited:
- WO-A1-2014/193895
- WO-A1-2015/042602
- US-A1- 2008 241 112
- US-A1- 2008 241 112
- US-A1- 2010 323 027
- US-A1- 2010 330 050
- US-A1- 2012 225 130
- US-A1- 2013 110 132
- DENIS ANGOULVANT ET AL: "Mesenchymal stem cell conditioned media attenuates in vitro and ex vivo myocardial reperfusion injury", JOURNAL OF HEART AND LUNG TRANSPLANTATION, vol. 30, no. 1, 1 January 2011 (2011-01-01), US, pages 95 - 102, XP055555853, ISSN: 1053-2498, DOI: 10.1016/j.healun.2010.08.023
- RUENN CHAI LAI ET AL: "Exosome secreted by MSC reduces myocardial ischemia/reperfusion injury", STEM CELL RESEARCH, vol. 4, no. 3, 1 May 2010 (2010-05-01), NL, pages 214 - 222, XP055555858, ISSN: 1873-5061, DOI: 10.1016/j.scr.2009.12.003
- JAE-HYUNG LEE ET AL: "Analysis of Transcriptome Complexity Through RNA Sequencing in Normal and Failing Murine Hearts", CIRCULATION RESEARCH, vol. 109, no. 12, 9 December 2011 (2011-12-09), US, pages 1332 - 1341, XP055558291, ISSN: 0009-7330, DOI: 10.1161/CIRCRESAHA.111.249433
- DANHUA LI ET AL: "Transcriptome Analysis Reveals Distinct Patterns of Long Noncoding RNAs in Heart and Plasma of Mice with Heart Failure", PLOS ONE, vol. 8, no. 10, 29 October 2013 (2013-10-29), pages e77938, XP055558201, DOI: 10.1371/journal.pone.0077938
- KASAHARA ET AL.: "Use of mesenchymal stem cell -conditioned medium to activate islets in preservation solution", CELL MED., vol. 5, no. 2-3., 14 May 2013 (2013-05-14), pages 75 - 81, XP055435944

## Description

### STATEMENT OF GOVERNMENTAL RIGHTS

This invention was made with government support under TR001 108 awarded by the National Institutes of Health. The Government has certain rights in the invention.

### FIELD OF THE DISCLOSURE

The present description relates generally to the fields of tissue and organ preservation and/or assessment.

### BACKGROUND OF THE DISCLOSURE

Tissue and organ failure is a global cause of death. In some instances, tissue engraftment or organ transplant is the preferred therapy to prevent tissue and organ failure. However, even when donor tissue and organs become available, they may not be "available" for transplantation, for example, because they are functionally compromised, or would become functionally compromised during ex vivo transport. For example, nearly 70% of hearts from donors who have consented to donation remain non-utilized because the hearts are functionally compromised or are logistically unsuitable for transport to the recipient within a four- to six-hour window of ischemia, after which heart function is compromised.

Changes in the flow of properly oxygenated blood flow to and from the heart and vascular tissue can result in significant "ischemic" injury to the heart, including death of heart tissue. Starving cardiac tissue of adequate oxygen as may occur during a myocardial infarction, certain surgical procedures, accidents, complicated births, and organ transplants including heart transplants may result in ischemic injury. Further, rapid introduction of oxygenated blood to ischemic cardiac tissue can result in reperfusion injury (I/R).

The incidence of primary graft dysfunction (PGD), defined as heart failure in the newly transplanted heart is as high as 34%, again related to the ischemic time during transport. Primary graft failure is the major cause of death from cardiac transplant in the first 30 days (3-8% mortality) as well as cumulatively for the life of the patient.

Current methods for preventing or at least minimizing ischemic and/or I/R injury include rapid intervention to restore lost blood flow, improvement in surgical techniques and instrumentation, and controlled cooling and warming of the heart before, during, and after potentially damaging cardiac invents and interventions.

It is an object of the present disclosure to mitigate and/or obviate one or more of the above deficiencies.

DENIS ANGOULVANT ET AL, "Mesenchymal stem cell conditioned media attenuates in vitro and ex vivo myocardial reperfusion injury", JOURNAL OF HEART AND LUNG TRANSPLANTATION, US, (20110101), vol. 30, no. 1, doi:10.1016/j.healun.2010.08.023, iSSN 1053-2498, pages 95 - 102, discloses that in vitro cell injury was significantly reduced by MSC, MSC CM and CsA. PI3K inhibitors significantly attenuated the protection afforded by MSC CM but not growth factor inhibitors. Ex vivo experimentation showed that MSC CM significantly reduced myocardial ischemia/reperfusion injury.

RUENN CHAI LAI ET AL, "Exosome secreted by MSC reduces myocardial ischemia/reperfusion injury", STEM CELL RESEARCH, NL, (20100501), vol. 4, no. 3, doi:10.1016/j.scr.2009.12.003, iSSN 1873-5061, pages 214 - 222 discloses that exosomes secreted by MSCs reduced infarct size in a mouse model of myocardial ischemia/reperfusion injury. Therefore, MSC mediated its cardioprotective paracrine effect by secreting exosomes.

JAE-HYUNG LEE ET AL, "Analysis of Transcriptome Complexity Through RNA Sequencing in Normal and Failing Murine Hearts", CIRCULATION RESEARCH, US, (20111209), vol. 109, no. 12, doi:10.1161/CIRCRESAHA.111.249433, iSSN 0009-7330, pages 1332 - 1341 discloses use of transcriptome profiles in hearts as a central issue to better understand cardiac physiology and disease.

### SUMMARY OF THE DISCLOSURE

In a first aspect there is provided a composition for use in the prevention or treatment of ischemic injury and/or reperfusion injury and/or injury to the heart comprising:
- at least a portion of a mesenchymal stem cell conditioned medium (MSC-CM), wherein the MSC-CM comprises medium conditioned by contact with MSCs derived from adipose tissue; and
- the MSCs have been treated under hypoxic conditions selected from less than 10% O₂, less than 5% O₂, or 1% O₂; and wherein the MSCs have been treated with TNF-alpha.

In certain embodiments, the at least a portion of MSC-CM comprises exosomes separated from the MSC-CM.

In a second aspect there is provided, a method of treating tissue comprising:
- contacting the tissue with a composition comprising at least a portion of MSC-conditioned medium (MSC-CM) for a treatment period, wherein the MSC-CM comprises medium conditioned by contact with MSCs derived from adipose tissue;

the MSCs have been treated under hypoxic conditions selected from less than 10% O₂, less than 5% O₂, or 1% O₂; and
wherein the MSCs have been treated with TNF-alpha;
wherein the tissue is cardiac tissue, and the tissue is comprised by an organ, wherein the organ is stored *ex vivo* or *ex-corporeal.*

In certain embodiments, the tissue is cardiac tissue comprised by a heart, and wherein the heart is an adult heart, an infant heart, or a neonatal heart.

In certain embodiments, the contacting step comprises perfusing the organ tissue with the composition for the treatment period.

In certain embodiments, the contacting step prevents, at least in part, ischemic damage in the tissue, thereby improving and/or preserving tissue function.

In certain embodiments, the contacting step prevents, at least in part, reperfusion damage in the tissue, thereby improving and/or preserving tissue function.

In certain embodiments, the method further comprises:
- analyzing the transcriptome of the contacted tissue; and
- comparing the transcriptome of the contacted tissue to a baseline transcriptome measured in a matched non-ischemic tissue, thereby evaluating the tissue.

In certain embodiments, the analyzing step comprises analysis of one or more of following genes: Lonrf1, Chd6, Rhobtb1, Wipf3, Raph1, Slc41a3, Per2, Colq, Cldn5, Timp3, Hlf, Per Bcl9, Apold1, Cys1, Wee1, Mthfd1l, Col5a3, Sorbs1, Spon2, Slc43a3, Clmp, Rbp1, Prickle Nfic, Slc6a6, Nxn, Gpcpd1, Tef, Podn, Mmp14, Smco4, Slc39a14, Eif5a2, Tm4sf1, Slc4a8 Polr2a, Best3, Acot1, Xdh, Id1, Usp2, Zbtb16, Sox4, Plcb4, Dusp18, 2210011C24Rik, Sex Gja5, Plcd3, Arntl, Hspa1b, Eepd1, Rcan1, Ppplr3a, Palld, Mylk4, Cobll1, Nppb, Plekho2, Sox7, Cry2, Tmem171, Vamp5, Dpy19l3, Dnajb1, Mrpl28, Fry, Flt1, Neurl3, Naca, Neb, Bmp4, Hif3a, Npc1, Phf5a, Ccrn4l, Lrrc52, Synpo2, Cntfr, Ppfia4, Inhbb, Acot11, Sh2d4a, Ciart, Dctpp1, Cipc, Naa60, Leo1, Rgs16, Sik3, Gm15417, Pik3r1, Gem, Slco5a1, Gng11, Wnk2, Fam107a, Arhgap20, Guk1, Mapk10, Nerpud1, Nme3, Zmiz1, Ubap2, Fosl2, Hyal1 Gbp5, Pdcd7, Jun, Hhipl1, Mcf2l, Cox6a1, Ptprm, Dvl3, Fam212a, Adh1, Smim20, Vwa1, Tmtc1, Hspala, Fxn, Fkbp2, Eda, Cdpf1, Cdc42bpa, Iigp1, Sorbs2, Lzts1, Clic5, Ctnnbip1 Actn1, Fmo2, Mid1ip1, Paqr6, Tmem37, Atf7ip, Fis1, Foxo3, Adamtsl4, S100a16, Tnf, Ncoa3, Sp2, Gas1, Vstm4, Unc119b, Cry1, Ptpn18, Lmo4, Rasl11a, Pcdh1, Irs1, Myeov2, Adora2a, Rreb1, Phf19, Rem1, Man2a1, Atp10d, Vamp8, Ttpal, Ucp2, Sertad1, Usp54, Ncor2, ler2, Dnal4, Bri3bp, Mbnl2, Prepl, Uqcr11, 2210407C18Rik, Epas1, Gngt2, Thra, Ptk2b, Hint2, Ubr2, Plcg2, Gimap1, Stk35, Ndufb9 and Wnt5b.

In certain embodiments, the tissue is evaluated as suitable for transplantation if expression of one or more of ARNT1, TNF, CLDN5, Col5a3, and Slc41a3 is the same or decreased relative to the baseline transcriptome and/or if expression of one or more of MTHFD1, LONRF1, RHOBTB1, Cycs1, Hhipl1, and FAM107a is the same or increased relative to the baseline transcriptome;
In certain embodiments, the tissue is evaluated as unsuitable for transplantation if expression of one or more of ARNT1, TNF, CLDN5, Col5a3, and Slc41a3 is increased relative to the standard and/or if expression of one or more of MTHFD1, LONRF1, RHOBTB1, Cycs1, Hhipl1, and FAM107a is decreased relative to the baseline transcriptome.

In certain embodiments, the transcriptomic profile of the tissue is determined before the contacting step, during the contacting step, and/or after the contacting step; and wherein the results of the comparison are used to determine further treatment of the tissue with the composition.

In certain embodiments, the results of the comparison are used to identify suitability of the tissue for transplant.

In certain embodiments, the method further comprises contacting the tissue with a composition comprising at least a portion of MSC-CM for a treatment period, and repeating comparison of the transcriptome of the contacted tissue to a baseline transcriptome measured in a matched set of tissue, wherein the tissue is evaluated as suitable for transplantation if, according to the repeated comparison, expression of one or more of ARNT1, TNF, CLDN5, Col5a3, and Slc41a3 is the same or decreased relative to the baseline transcriptome and/or if expression of one or more of MTHFD1, LONRF1, RHOBTB1, Cycs1, Hhipl1, and FAM107a is the same or increased relative to the baseline transcriptome.

### DESCRIPTION OF THE DRAWINGS

The patent or application file contains at least one drawing in color. Copies of this patent or patent application publication with color drawings will be provided by the Office upon request and payment of the necessary fee.

These and other features of the disclosure will become more apparent in the following detailed description in which reference is made to the appended drawings wherein:
**FIG. 1** depicts graphs illustrating the baseline cardiac function in hearts measured 7, 8, 10, and 11 days after birth.
**FIG. 2** depicts graphs illustrating the effect on post-ischemic myocardial function in neonates pretreated with ASC-CM , measured 7-1 1 days after birth.
**FIG. 3** depicts graphs illustrating the effects of pretreatment with ASC-CM on recovery of post ischemic myocardial function in neonates 7-11 days after birth.
**FIG. 4** depicts gels showing p-STAT3 and T-STAT3 levels present in control medial and in ASC-CM media; graph illustrating the relative level of p-STAT3 /T-STAT3 (%) determined in media c and in ASC-CM .
**FIG. 5** depicts gels showing procaspase-3, active caspase-3 p17, and GAPDH levels present in control medial and in ASC-CM media; graph illustrating the level of active caspase-3 p17 as a percentage of procaspase-3 in media c and in ASC-CM .
**FIG. 6** **depicts a** graph illustrating myocardial cytokine production after I/R injury in neonates 7-1 1 days after birth.
**FIG. 7** depicts a cartoon showing some of the steps in treating adult hearts with an infusion of ASC-CM .
**FIG. 8** depicts graphs illustrating the levels of LVDP as a percent of Eq measured before ischemia, during ischemia, and during reperfusion of adult hearts.
**FIG. 9** depicts a cartoon showing some of the steps in treating pediatric hearts with an infusion of ASC-CM .
**FIG. 10** depicts graphs illustrating the levels of LVDP as a percent of Eq measured at Eq, during ischemia, and during reperfusion measured among different ages of neonatal and infant hearts following I/R.
**FIG. 11** depicts graphs illustrating the levels of LVDP as a percent of Eq measured during Eq., during ischemia, and during reperfusion in neonates and infants 8 days and 22 days after birth, with or without pretreatment of ASC-CM .
**FIG. 12** depicts graphs illustrating the levels of LVDP as a percent of Eq measured during Eq., during ischemia, and during reperfusion measured in neonatal and infant hearts at 7 days after birth.
**FIG. 13** depicts graphs showing gene expression during ischemia (blue) and progressive restoration towards their normal non-ischemic levels ("1") by Ad-MSC CM (conditioned media) (Ischemia/CM , red) and Ad-MSC CM obtained during hypoxia (H-CM, green). Genes decreased by ischemia and progressively normalized by CM and then by hypoxic-CM . Fold vs. control, which is "1".
**FIG. 14** depicts graphs showing gene expression during ischemia (blue) and progressive restoration towards their normal non-ischemic levels (purple) by Ad-MSC CM (Ischemia/CM, red) and Ad-MSC CM obtained during hypoxia (H-CM, green). Genes decreased by ischemia and progressively normalized by CM and then by hypoxic-CM. Fold vs. control, which is "1".
**FIG. 15** depicts graphs showing gene expression during ischemia (blue) and progressive restoration towards their normal non-ischemic levels ("1") by Ad-MSC CM (Ischemia/CM , red) and Ad-MSC CM obtained during hypoxia (H-CM, green); multiple TNF pathway genes are increased in ischemia and normalized by ASC secretome.
**FIGS. 16A-E** illustrate preservation of the normal transcriptomal fingerprint in: FIG. 16A complex I of the mitochondrial electron transport chain; FIG. 16B complex III of the mitochondrial electron transport chain; FIG. 16C complex IV of the mitochondrial electron transport chain; FIG. 16D complex V of the mitochondrial electron transport chain; FIG. 16E mitochondrial ribosomal family.
**FIG. 17** depicts a graph showing component analysis based on linear combination of six genes increased by ischemia (Y axis) and six genes decreased by ischemia (X axis).
**FIG. 18** depicts a graph showing component analysis based on linear combination of six genes increased by ischemia (Y axis) and six genes decreased by ischemia (X axis).
**FIG. 19** shows that Ad-MSC derived CM returns transcriptional fingerprint towards normal in almost every gene, by about 75%.

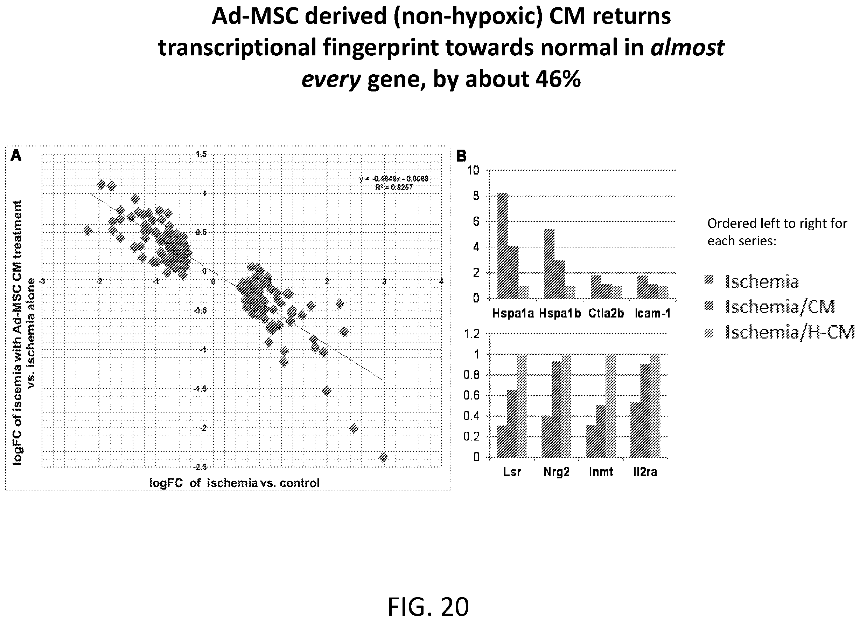

**FIG. 20** shows that Ad-MSC CM potently restores myocardial transcriptome profiling towards non-ischemic control levels. A. The log FC (fold change) of expression in untreated ischemic hearts vs. normal non-ischemic hearts (control) is plotted on the X-axis, and the log FC of expression in Ad-MSC CM-treated ischemic hearts vs. untreated ischemic hearts. The transcriptional "fingerprint" demonstrates restoration towards normal, reflected by the negative (-0.46) slope of the correlation line. The correlation coefficient is 0.83. B. Six-hour ischemia up- and down-regulated representative genes. The expression level of each gene is plotted as a ratio to the non-ischemic control value, set at one.
**FIG. 21** shows that Ad-MSC derived CM returns transcriptional fingerprint towards normal in almost every gene, by 46% with CM secreted in normoxia and by 75% with CM secreted in hypoxia.

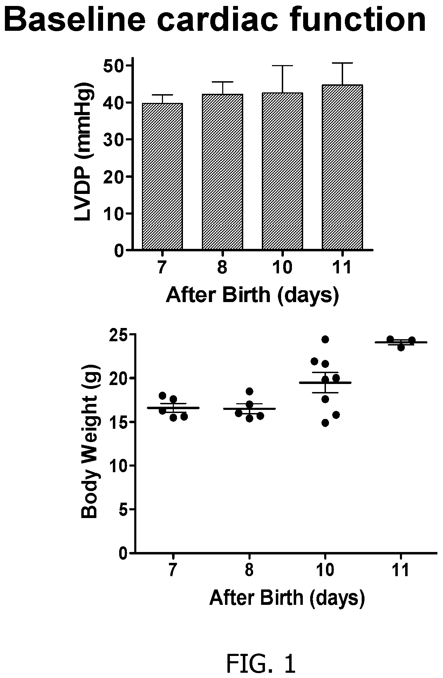

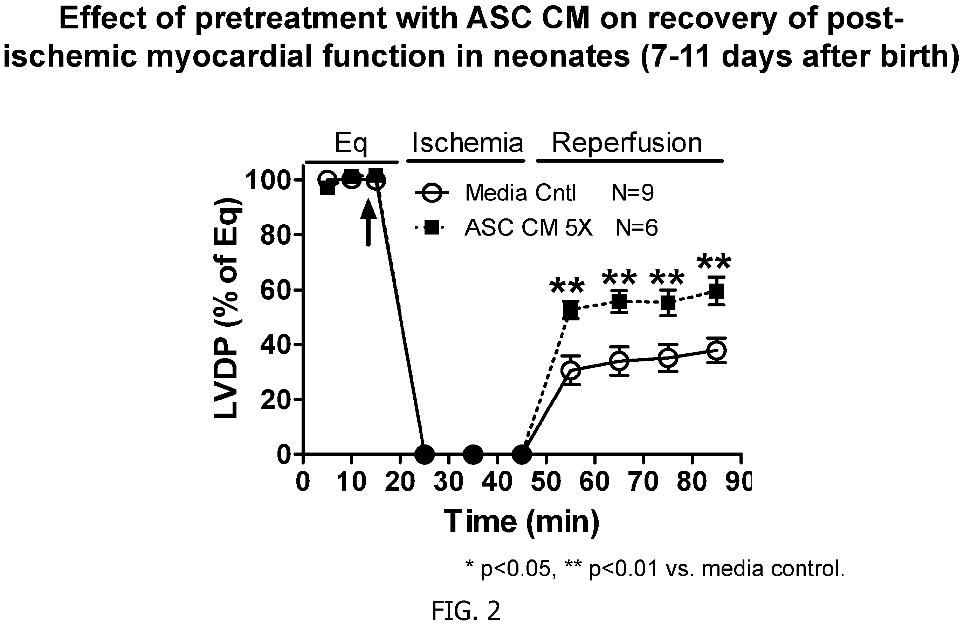

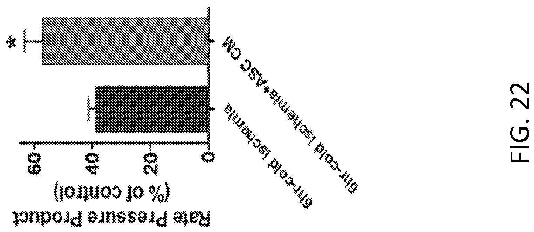

**FIG. 22** shows that concentrated CM from Ad-MSC preserved function of isolated mouse hearts during conditions commonly used to transport human donor hearts. Mouse hearts were isolated from mice and immediately perfused on the Langendorff apparatus with 1 ml of UW solution either without or containing CM from Ad-MSC. (Final concentration of the CM was 8x original). The hearts (N=5 each group) were then placed in a tube containing 0.5 ml of the same solution and incubated at 4 °C for 6 hours. Finally, hearts were evaluated on the Langendorff system for their functional response to ischemia and reperfusion as in Figs. 1 and 2, except the hearts were allowed to contract spontaneously. Therefore the values are expressed as rate-pressure product (RPP) = spontaneous rate x left-ventricular developed pressure (LVDP).

### DETAILED DESCRIPTION OF THE DISCLOSURE

For the purposes of promoting an understanding of the principles of the novel technology, reference will now be made to the preferred embodiments thereof, and specific language will be used to describe the same. It will nevertheless be understood that no limitation of the scope of the novel technology is thereby intended, such alterations, modifications, and further applications of the principles of the novel technology being contemplated as would normally occur to one skilled in the art to which the novel technology relates are within the scope of this disclosure and the claims.

Unless defined otherwise, all technical and scientific terms used herein have the same meaning as commonly understood by one of ordinary skill in the art to which this disclosure belongs.

### General Description of Methods and Compositions for Tissue Treatment

Mitigating and/or preventing ischemic injury and/or reperfusion injury is important for recovery of tissue and/or organ function in patients undergoing engraftment or transplantation. As described herein, the inventors have discovered that compositions comprising a secretion from mesenchymal stem cells (MSCs) and/or at least a portion of mesenchymal stem cell-conditioned medium (MSC-CM), also referred to herein as "MSC compositions", can be used to preserve (at least in part) and/or rescue (at least in part) tissue from ischemic and/or reperfusion injury. MSCs, also known as mesenchymal stromal cells, can effect in a tissue one or more of inflammation, reactive oxygen species, apoptosis, angiogenesis, and proliferation of tissue-endogenous stem-like cells. MSCs secrete a plurality of paracrine factors, such as cytokines, chemokines, and factors that regulate in a tissue one or more of apoptosis, inflammation, immunity, and angiogenesis. In a preferred embodiment, the MSCs are derived from adipose tissue (Ad-MSCs) . In a particularly preferred embodiment, the MSC composition comprises at least a portion of a medium conditioned with the MSCs (i.e., MSC conditioned medium (MSC-CM), such as Ad-MSC conditioned medium (Ad-MSC-CM)).

The materials and methods provided herein are applicable to a variety of tissues and organs, in a variety of functional states (e.g., abnormal tissue/organ function, such as impaired function). For example, tissues and organs characterized by being susceptible to ischemia and hypoxia-induced progressive cell damage are suitable for use with the materials and methods provided herein. For example, it is contemplated that cardiac tissue, (claimed) lung tissue, pancreatic tissue, skin, cartilage, bone, and/or cornea (not claimed) tissue are suitable for use with the materials and methods provided herein. For example, it is contemplated that hearts,(claimed) kidneys, lungs, livers, hands, feet, and/or faces (not claimed) are suitable for use with materials and methods provided herein. Further, it is contemplated that tissues and/or organs obtained from adults, infants and/or neonatals are suitable for use with the materials and methods provided herein.

As described further below, in a preferred embodiment, perfusion of Ad-MSC-CM into adult, infant, or neonatal hearts protects the hearts (at least in part) from loss of function caused by ischemic injury and/or reperfusion injury. At least some of the compounds Ad-MSCs secrete into the cell culture medium provide a protective and/or restorative effect on adult, infant, and neonatal cardiac tissue.

As discussed below, various concentrations of Ad-MSC-CM can be used to treat human or animal patients before or after the patient undergoes an ischemic event. It is also contemplated that the compositions provided herein can be used to reduce or prevent reperfusion damage to adult, infant, or neonatal cardiac tissue. Pre-treatment of the MSCs used to condition the MSC-CM may also improve treatment of cardiac tissue before, during, and/or after an ischemic and/or reperfusion event. Therapeutic benefit of the disclosed method and composition may be measured and/or monitored by way of functional assays and/or molecular assays, such as the transcriptomic profiling provided herein. Further, the transcriptomic profiling disclosed herein may be used to evaluate tissues and/or organs to determine the presence, absence, and/or level of ischemic and/or reperfusion damage.

### MSC Compositions

In an aspect, the compositions provided herein comprise MSC secretions and/or MSC-conditioned medium (MSC-CM) suitable for use in prevention or treatment of ischemic injury and/or reperfusion injury and/or injury to the heart.

MSCs can be obtained for various sources, such as, for example, bone marrow (BM), peripheral blood, adipose tissue, placenta, umbilical cord, or from pluripotent stem cells, such as embryonic stem cells, fetal stem cells, adult stem cells, or induced pluripotent stem cells (iPSCs). In a preferred embodiment, the MSCs are obtained from adipose tissue.

Adipose-derived MSCs, also referred to herein as adipose stem cells (ASCs), are present in adult and pediatric adipose tissue. Ad-MSCs can be cultured in a cell culture medium, *in vitro.* After a period of time in culture, the Ad-MSCs can be separated from the medium, and the medium collected. This medium, conditioned with Ad-MSCs, is referred to as Ad-MSC-conditioned medium (Ad-MSC-CM), and it contains various components secreted by the Ad-MSCs during the period of culture time.

The factors secreted by MSCs, also referred to as the secretome, may include microvesicles, extracellular vesicles (EVs) and/or the MSCs exosome, and can be found in the cell culture medium where the MSCs are cultured. This medium, conditioned by exposure to MSCs, is referred to herein as conditioned medium (CM). Medium may be conditioned with MSCs for a range of times to obtain suitable MSC-CM, such as, for example, between about 20 minutes to 96 hours, 20 minutes to 72 hours, 20 minutes to 60 hours, 20 minutes to 48 hours, 20 minutes to 36 hours, 20 minutes to 24 hours, 20 minutes to 12 hours or 20 minutes to 6 hours. Compositions suitable for use with the disclosed method may comprise all or a portion of MSC-CM . For example, MSC-CM may be perfused into a tissue, without further treatment to alter the composition of the MSC-CM . Alternatively, the MSC-CM may be diluted, concentrated, or separated to obtain a specific portion of the CM, or combined with one or more other compounds or compositions, such as, for example a solution for transporting and/or preserving an organ (e.g., UW solution, Stanford solution, Steen solution etc.). In an aspect, the MSC compositions provided herein may be used as an adjunct to an organ transport/preservation solution.

In a preferred embodiment, the composition provided herein comprises EVs separated from MSC-CM . EVs contain cargos of factors that may be unstable in the extracellular milieu, such as microRNAs. In a particularly preferred embodiment, the composition provided herein comprises exosomes separated from MSC-CM . In an embodiment, it is contemplated that a composition suitable for use in the disclosed method comprises exosomes separated from MSC-CM , such as, for example, Ad-MSC-CM . An MSC composition comprising exosomes may be beneficial, at least because, relative to an MSC composition comprising all the contents of and MSC-CM , its composition may be easier to define, standardize, assay for toxicity, and/or store for a time period (e.g., improved shelf life).

In various embodiments, the MSCs used to condition the CM may be "preconditioned" with one or more treatments. By preconditioned, we mean exposed to a treatment, such as, for example, an environmental condition, one or more small molecules and/or proteins.

In a preferred embodiment, the MSCs used to condition the CM are exposed to hypoxic conditions (e.g., less than 10% O₂, less than 5% O₂, or about 1% O₂). Hypoxia may be induced in the MSC cell culture in a variety of ways, such as, for example, by altering the gas composition the cells are exposed to or by providing one or more chemical inducer of hypoxia to the MSCs in culture.

In a preferred embodiment, the MSCs used to condition the CM are exposed to TNF-alpha.

In an embodiment, a tissue preparation suitable for transplantation is provided. The tissue preparation comprises an organ or a segment thereof and an MSC composition, as described herein. Contact between the tissue preparation and the MSC composition protects (at least in part) and/or reverses (at least in part) ischemic and/or reperfusion injury of the tissue, thereby preparing the tissue such that it is suitable for transplantation.

### Method of Treating Tissue with MSC Compositions

In an aspect, an ex vivo method of treating a tissue with an MSC composition is provided. By treating, we mean preventing and/or mitigating, at least in part, ischemic and/or reperfusion injury of the tissue or rescuing, at least in part, the tissue from ischemic and/or reperfusion injury. For example, a tissue may be perfused with an MSC composition disclosed herein, for a period of time, thereby preventing or mitigating ischemic and/or reperfusion injury of the tissue or rescuing the tissue from ischemic and/or reperfusion injury. Various systems for perfusing tissues and organs are known, such as, for example, the Langendorff system or a tissue/organ bath system.

in an embodiment, the tissue may be treated ex vivo. For example, in various organ transplant systems, the donor organ is maintained ex *vivo* for a period of time. During this time, there is inadequate blood flow to the organ, and consequently inadequate oxygen supply to the organ. This period of ischemia (also referred to herein as an ischemic event) damages the organ, as discussed above. When blood supply returns to the tissue (i.e., reperfusion), after the ischemic event, it can injure the tissue, for example by causing inflammation and oxidative stress, rather than restoring normal tissue function. Cardioplegia is another example in which an organ undergoes a period of ischemia and is suitable for treatment with the method provided herein.

In an embodiment, the compositions are for use in treating ischemic injury and/or reperfusion injury and/or injury to the heart. For example, during myocardial infarction the heart undergoes ischemia, which damages cardiac tissue.

in various aspects of the method provided, perfusion of tissue with an MSC composition may be carried out before, during and/or after an ischemic event. For example, in an effort to recover a heart from a brain-dead donor, the heart may be treated using the method provided herein prior to cold ischemia. For example, in an effort to recover a heart donated after circulatory death, the heart may be treated using compositions for use as disclosed after a period of warm *(in situ)* ischemia. For example, a patient undergoing or having recently undergone myocardial infarction may be treated using compositions for use as disclosed. In an embodiment, treatment may be systemic, wherein the MSC composition is provided to the patient systemically, or local, wherein the MSC composition is provided locally to the heart such as, for example, by way of intracoronary delivery or retrograde venous infusion. Alternatively or additionally, in an embodiment, the perfusion may be carried out before and/or during reperfusion.

Perfusion with a suitable MSC composition, as provided herein, may be carried out at various doses over various time periods. For example, and MSC concentration may be provided in a range of about 1X-1 0X before, during and/or after ischemia. In various embodiments, the MSC composition is provided as an adjunct to treatment with an organ transport/preservation solution, such as UW solution, Stanford solution, Steen solution, etc.

Results of tissue treatment with a suitable MSC composition, as provided herein, may be measured in a variety of ways, such as, for example, by functional assay (i.e., to determine one or more indicator of tissue/organ function), or molecular assay (i.e. , to determine one or more molecular feature of the tissue/organ) .

In one embodiment, one or more functional assay is used to determine results of the treatment, wherein results of the functional assay are compared to a standard. For example, the standard for a functional assay may be indicative or a normally functioning tissue/organ , or an abnormally functioning tissue/organ (e.g., a tissue/organ having impaired function).

In one embodiment, one or more molecular assay is used to determine results of the treatment, wherein results of the molecular assay are compared to a standard. For example, the standard for a molecular assay may be indicative or a normally functioning tissue/organ , or an abnormally functioning tissue/organ (e.g., a tissue/organ having impaired function).

In one preferred embodiment, the molecular assay is a transcriptomic assay, used to determine a level of transcript expression of a plurality of genes.

### Transcriptomic Evaluation of Tissue

The inventors have determined a gene expression profile, also referred to herein as a "transcriptomic profile", or a "fingerprint", for normal cardiac tissue. The inventors have also determined that cardiac tissues having various levels of ischemic damage have transcriptomic profiles that differ from the normal cardiac transcriptomic profile.

It is contemplated that as tissue becomes progressively ischemic, its transcriptome will vary to a greater degree from normal. Such variance, may be in proportion (although not necessarily linear proportion) to the degree and time of ischemia, and in proportion to the functional damage to the tissue, It is contemplated that this relationship between ischemic damage and transcriptome profile variance will present in warm ischemia, such as that sustained within the body upon inadequate blood flow or circulatory death (e.g., myocardial infarction). It is also contemplated that this relationship between ischemic damage and transcriptome profile variance will present in cold ischemia, such as that sustained by an organ that is removed from the body of a donor in order to transport it to a recipient.

The inventors have found that in the context of cold ischemia, conducted in the presence of prior perfusion with a standard preservation solution, that there is a progressive alteration in the transcriptomic profile of cardiac tissue, at 0, 2 and 6 hours of ischemia. Under these circumstances, using a statistical stringency criterion of false discovery rate less than .05, only 184 of over 13,000 individual RNA transcripts tested were statistically detectably different between 0 and 6 hours of ischemia (0 hours of ischemia being a "standard" or "normal") . It is contemplated that this relatively low number of transcripts deviating statistically significantly from normal is a consequence of the cooling and preservation treatments.

The 184 differentially expressed genes included: Lonrf1 , Chd6, Rhobtbl , Wipf3, Raphl , Slc4 1a3, Per2, Colq, Cldn5, Timp3, Hlf, Per3, Bcl9, Apold I , Cys1 , Wee1 , Mthfd I ₗ, Col5a3, Sorbsl , Spon2, Slc43a3, Clmp, Rbp1 , Prickle3, Nfic, Slc6a6, Nxn, Gpcpd I , Tef, Podn, Mmp1 4, Smco4, Slc39a1 4, Eif5a2, Tm4sf1 , Slc4a8, Polr2a, Best3, Acotl , Xdh, Id 1, Usp2, Zbtbl 6, Sox4, Plcb4, Duspl 8, 221 001 1C24Rik, Sex, Gja5, Plcd3, Arntl, Hspal b, Eepd I , Rcan I , Ppp1 r3a, Palld, Mylk4, CobiM , Nppb, Plekho2, Sox7, Cry2, Tmem 171, Vamp5, Dpy1 9l3, Dnajbl , Mrpl28, Fry, Flt1 , Neurl3, Naca, Neb, Bmp4, Hif3a, Npc1 , Phf5a, Ccrn4l, Lrrc52, Synpo2, Cntfr, Ppfia4, Inhbb, Acotl 1, Sh2d4a, Ciart, Dctppl , Cipc, Naa60, Leo1 , Rgs1 6, Sik3, Gm1541 7, Pik3r1 , Gem, Slco5a1 , Gng 11, Wnk2, Fam 107a, Arhgap20, Guk1 , Mapk1 0, Herpud I , Nme3, Zmizl , Ubap2, Fosl2, HyaM , Gbp5, Pdcd7, Jun, HhipM , Mcf2l, Cox6a1 , Ptprm, Dvl3, Fam2 12a, Adh 1, Smim20, Vwa1 , Tmtc1 , Hspal a, Fxn, Fkbp2, Eda, Cdpfl , Cdc42bpa, ligp1 , Sorbs2, Lztsl , Clic5, Ctnnbipl , Actn I , Fmo2, Mid 1ᵢₚ₁ , Paqr6, Tmem37, Atf7ip, Fis1 , Foxo3, Adamtsl4, S 100a1 6, Tnf, Ncoa3, Sp2, Gas1 , Vstm4, Unci 19b, Cry1 , Ptpn 18, Lmo4, Rash 1a, Pcdh i , Irs1 , Myeov2, Adora2a, Rrebl , Phf1 9, Rem1 , Man2a1 , Atp1 0d, Vamp8, Ttpal, Ucp2, Sertad I , Usp54, Ncor2, ler2, Dnal4, Bri3bp, Mbnl2, Prepl, Uqcr1 1, 221 0407C1 8Rik, Epasl , Gngt2, Thra, Ptk2b, Hint2, Ubr2, Plcg2, Gimapl , Stk35, Ndufb9, Wnt5b.

For example, genes whose expression was decreased by ischemia include: redox regulated tumor suppressor genes; MTHFD1 , a cytosolic trifunctional THF synthese and NADPH producer that is frequently deleted in cancer (ID: 4522 for human and ID: 1081 56 for mouse); LONRF1 , an ATP-dependent SOS protease that is frequently deleted in cancer (ID: _{9 1} 694 for human and ID: 244421 for mouse); RHOBTB1 , a member of the rho family of GTPases needed for actin cytoskeleton associated signaling, which is frequently deleted in cancer (ID: 9886 for human and ID: 69288 for mouse); Cycsl , a cilia associated gene that is lost in polycystic kidney disease, regulated by thiol redox and myristoylation and binds to IGF1 ; HhipM , a quinone oxidoreductase, regulated by thiol redox (abnormalities in this gene confer heart disease risk (ID: 9886 for human and ID: 69288 for mouse.); and FAM 107a, also called TU3A, which is often deleted in renal cell cancer (ID: 9886 for human and ID: 69288 for mouse).

For example, genes whose expression was increased by ischemia include: redox, pH, and catabolite activated cell danger response genes, such as ARNT1 , aryl hydrocarbon nuclear transpoter, which facilitates tryptophan metabolite activated cell danger signalling (ID: 405 for human); Spon2, spondin 2, an extracellular matrix protein (ID: 1041 7 for human and ID: 100689 for mouse); TNF, tumor necrosis factor, a classic cell danger/innate immunity signaling molecule (ID: _{7 1} 24 for human and ID: 2 1926 for mouse); CLDN5, claudin5, a classic cell danger response suppressor, tight junction protein, which is frequently deleted in the autism syndrome velo-cardial-facial syndrome (ID: _{7 1} 22 for human and ID: 12741 for mouse); Col5a3, collagen 5a3 fibrillar collagen gene, which is critical for heart structural maintenance (ID: 50509 for human and ID: 53867 for mouse); and Sic41 a3, which is a magnesium transporter needed to maintain intracellular Mg2+ for bioenergetics during glycolysis (ID: 54946 for human and ID: _{7 1} 699 for mouse).

In an embodiment, transcriptomic profiles of various gene families are representative of ischemic, non-ischemic, and/or recovered tissues, such as, for example, the TNF family of genes, complex I, III, IV and/or V of the mitochondrial electron transport chain, and/or the mitochondrial ribosomal family of genes.

When hearts were pre-treated with Ad-MSC-CM or with Ad-MSC-CM generated by pre-treating the Ad-MSCs under hypoxic incubation, there was less deviation of transcript expression from normal. Specifically, after normoxic Ad-MSC-CM treatment, approximately 14 transcripts were detectably different from a normal transcriptomic profile. Following hypoxic Ad-MSC CM treatment, only one transcript was detectably different from a normal transcriptomic profile. A person skilled in the art will appreciate that the specific number of transcript variants may vary with experimental condition and/or with the selection of statistical stringency criteria. However, the data provided herein indicate that the functional deterioration of tissue, which is associated with hypoxic time, is concurrent with deviation of the tissue's transcriptomic fingerprint relative to a normal tissue. Further, the progressive restoration of tissue function that occurred with Ad-MSC CM exposure occurred in conjunction with preservation of a normal transcriptome, to at least some degree.

### Method of Evaluating and/or Monitoring Tissues and/or Organs

In one embodiment, the methods include evaluating a tissue or organ for ischemic damage is provided. The method involves providing a sample of a tissue, analyzing at least a portion of the transcriptome of the sample, and comparing the analyzed portion of the transcriptome to a standard.

In an embodiment, the method involved analyzing the expression of transcripts of one or more of the following genes: Lonrfl , Chd6, Rhobtbl , Wipf3, Raph I , SIc41 a3, Per2, Colq, Cldn5, Timp3, Hlf, Per3, Bcl9, Apold I , Cys1 , Wee1 , Mthfd i ₗ, Col5a3, Sorbsl , Spon2, Slc43a3, Clmp, Rbp1 , Prickle3, Nfic, Slc6a6, Nxn, Gpcpd I , Tef, Podn, Mmp1 4, Smco4, SIc39a1 4, Eif5a2, Tm4sf1 , Slc4a8, Polr2a, Best3, Acotl , Xdh, Id 1, Usp2, Zbtb1 6, Sox4, Plcb4, Dusp1 8, 221 001 1C24Rik, Sex, Gja5, Plcd3, Arntl, Hspal b, Eepd I , Rcan i , Ppp1 r3a, Palld, Mylk4, CobIM , Nppb, Plekho2, Sox7, Cry2, Tmem 171, Vamp5, Dpy1 9l3, Dnajbl , Mrpl28, Fry, Flt1 , Neurl3, Naca, Neb, Bmp4, Hif3a, Npc1 , Phf5a, Ccrn4l, Lrrc52, Synpo2, Cntfr, Ppfia4, Inhbb, Acotl 1, Sh2d4a, Ciart, Dctppl , Cipc, Naa60, Leo1 , Rgs1 6, Sik3, Gm1541 7, Pik3r1 , Gem, Slco5a1 , Gng 11, Wnk2, Fam 107a, Arhgap20, Guk1 , Mapkl _{O}, Herpud I , Nme3, Zmizl , Ubap2, Fosl2, HyaM , Gbp5, Pdcd7, Jun, HhipM , Mcf2l, Cox6a1 , Ptprm, Dvl3, Fam21 2a, Adh 1, Smim20, Vwa1 , Tmtc1, Hspa I ₐ, Fxn, Fkbp2, Eda, Cdpfl , Cdc42bpa, ligp1 , Sorbs2, Lztsl , Clic5, Ctnnbipl , Actn I , Fmo2, Mid 1ip 1, Paqr6, Tmem37, Atf7ip, Fis1 , Foxo3, Adamtsl4, S 100a1 6, Tnf, Ncoa3, Sp2, Gas1 , Vstm4, Unci 19b, Cry1 , Ptpn 18, Lmo4, Rash 1a, Pcdh i , Irs1 , Myeov2, Adora2a, Rrebl , Phf1 9, Rem1 , Man2a1 , Atpl Od, Vamp8, Ttpal, Ucp2, Sertad I , Usp54, Ncor2, ler2, Dnal4, Bri3bp, Mbnl2, Prepl, Uqcr1 1, 221 0407C 18Rik, Epasl , Gngt2, Thra, Ptk2b, Hint2, Ubr2, Plcg2, Gimapl , Stk35, Ndufb9 and Wnt5b. In a preferred embodiment, the method involves analyzing the expression of one or more of: MTHFD1 , LONRF1 , RHOBTB1 , Cycsl , HhipM , FAM 107a, ARNT1 , Spon2, TNF, CLDN5, Col5a3 and SIc41 a3.

In an embodiment, evaluating a tissue or organ may be used to determine the extent of ischemic injury in a tissue/organ.

In an embodiment, evaluating a tissue or organ may be used to determine the level of efficacy of a method of treating the tissue/organ with an MSC composition, as provided herein.

The methods disclosed may be for evaluating organs for transplant is provided. The method involves analyzing the transcriptome of an organ stored in vitro in a transplant buffer for at least 2 hours and comparing the transcriptome of the organ to a baseline transcriptome measured in a matched set of organs immediately after harvesting.

In an embodiment, the organ is evaluated as suitable for transplantation if expression of one or more of ARNT1 , TNF, CLDN5, Col5a3, and Sic41 a3 is the same or decreased relative to the baseline transcriptome and/or if expression of one or more of MTHFD1 , LONRF1 , RHOBTB1 , Cycsl , HhipM , and FAM 107a is the same or increased relative to the baseline transcriptome.

In an embodiment, the organ is evaluated as unsuitable for transplantation if expression of one or more of ARNT1 , TNF, CLDN5, Col5a3, and Sic41 a3 is increased relative to the standard and/or if expression of one or more of MTHFD1 , LONRF 1, RHOBTB1 , Cycsl , HhipM , and FAM 107a is decreased relative to the baseline transcriptome.

In an embodiment, the method further involves contacting the organ with a composition comprising at least a portion of MSC-CM for a treatment period, and repeating comparison of the transcriptome of the organ to a baseline transcriptome measured in a matched set of organs immediately after harvesting.

Accordingly, in various embodiments, a tissue or organ may be assayed using one or more functional and/or molecular assays, to evaluate its status (e.g., extent of ischemic and/or reperfusion damage, and/or suitability for transplantation), before, during, and/or after treatment with the method provided herein. In this way, a practitioner can monitor the health of a tissue or organ, for example, during ex vivo transport, during treatment, post-treatment, and/or over the course of transplantation. Monitoring may allow a practitioner to determine if the tissue or organ is suitable for transplantation.

### Kits

Disclosed but not claimed are kits for carrying out the methods disclosed herein. Such kits typically comprise two or more components required for treatment of a tissue or organ, as provided herein. Components of the kit include one or more MSC compositions, and one or more of compounds, reagents, containers, equipment, and instructions for using the kit. Accordingly, the methods described herein may be performed by utilizing pre-packaged kits provided herein. In an example not claimed, the kit comprises one or more MCS composition and instructions. In an example not claimed , the instructions comprise one or more protocols for preparing and/or using the MSC composition in the method provided herein. In examples not claimed , the kit comprises primers and reagents for analyzing at least a portion of a tissue's transcriptomic profile and instructions comprising one or more protocols for analyzing the transcriptome, such as, for example, instructions for comparison to one or more standards. In examples not claimed , the kit comprises one or more standards (e.g., standard comprising a biological sample, or representative transcript expression data).

In examples not claimed , the kit comprises MSC-CM , as described herein. By way of example, the kit may contain a container comprising one or more doses of MSC-CM and instructions for using the MSC-CM . In examples not claimed , the kit may further comprise one or more organ transplant/preservation composition, such as UW solution, Stanford solution, Steen solution etc.

In examples not claimed, the kit comprises Ad-MSC-CM , as described herein. By way of example, the kit may contain container comprising one or more doses of Ad-MSC-CM and instructions for using the Ad-MSC-CM . In a preferred embodiment, the kit may comprising a composition comprising exosomes separated from Ad-MSC-CM .

The following examples illustrative of the disclosure are provided. Benefits of perfusion with adipose-derived mesenchymal stem cells (Ad-MSCs) and/or Ad-MSC conditioned medium (Ad-MSC-CM) using cardiac rodent models are demonstrated.

### Example 1: Treatment of Cardiac Tissue with MSC-CM Prior to- and Post-ischemia

### METHODS

### Isolation of Ad-MSCs and Ad-MSC-CM

The cells were obtained from frozen stocks of cells, cultured from human lipoaspirate samples, which were previously collected in the inventor's laboratory within an IRB-approved protocol. Samples were selected from three healthy adult donors aged 21-45. Prior to use, Ad-MSC were routinely analyzed with fluorescent activated cell sorting (FACS) and appropriate differentiation potential demonstrated to confirm cellular characteristics, as harmonized by multiple groups, and accepted by the International Society for Cellular Therapy.

Conditioned medium was generated from the same Ad-MSCs. Additional Ad-MSCs were isolated from abdominal subcutaneous fat of two additional healthy human female donors. All isolations were conducted using standard methods. Ad-MSC cell stocks had been frozen at passages 2 and 3 and had been phenotypically characterized based on cell surface markers of MSCs (CD1 0, CD1 3, CD29, CD73, CD44).

From each donor, Ad-MSC CM was generated by incubating 10 ml of serum- free medium over an Ad-MSC monolayer (75 cm², at 4×10⁴ cells/cm² or equivalent) for 72 hours, and was concentrated, as described previously by centrifugation through Amicon Ultra Centrifugal Filter units with membranes selective for >3 kDa (EMD Millipore).

### Ex vivo Perfusion of Isolated Beating Mouse Hearts (Langendorff Protocol)

Hearts were isolated from adult male C57BL/6 mice. Briefly, after mice were anesthetized and heparinized, mouse hearts were rapidly excised and placed in 4°C K-H solution. The aorta was cannulated immediately on the Langendorff apparatus and a three-way stopcock above the aortic root was used to infuse treatment reagents or create global ischemia. LVDP and the maximal positive and negative values of the first derivative of pressure (+dP/dt and -dP/dt) were recorded using a PowerLab 8 preamplifier/digitizer (AD Instruments Inc., Milford, MA). Coronary flow was measured by collecting pulmonary artery effluent. After recording the baseline LV function, the heart was infused with hyperkalemic cardioplegia (15 mM K+ in University of Wisconsin [UW] solution) and stored in UW solution at 37°C for 20 min or at 4°C for six hours. The efficacy of each type of MSC as well as their CM was tested by re-suspending each in UW solution and injecting into the coronary circulation.

### Treatment of cardiac tissue with MSC-CM prior to ischemia

Referring now to FIG. 1, baseline cardiac function in hearts was measured at 7, 8, 10, and 11 days after birth.

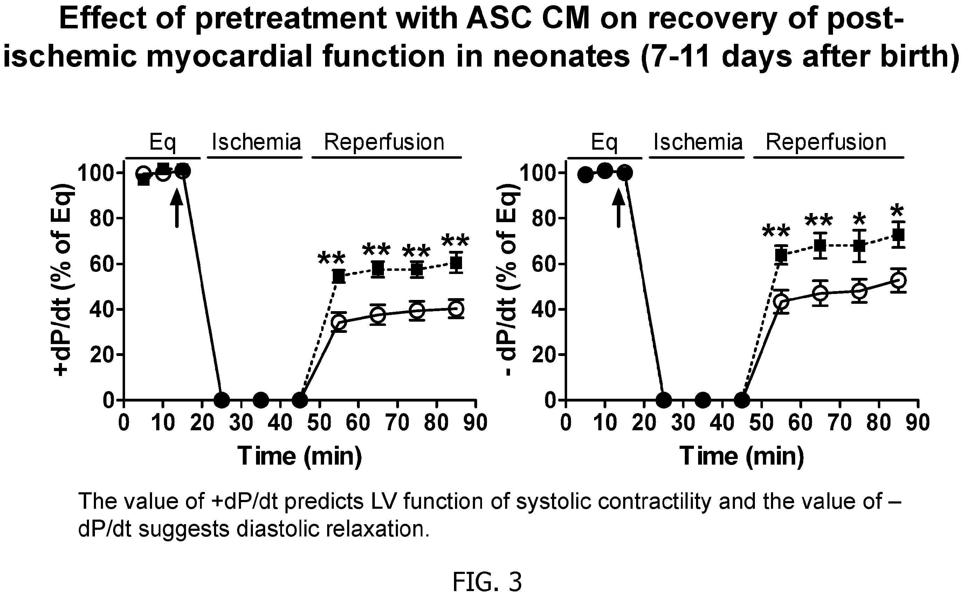

Referring now to FIGS. 2 and 3, ASC-CM was delivered into the myocardium via coronary infusion before global ischemia of isolated rat hearts (Langendorff). Functional recovery was determined during reperfusion. Pre-treatment of cardiac tissue with ASC-CM significantly improved post-ischemic myocardial functional recovery in neonatal rat hearts following global I/R injury.

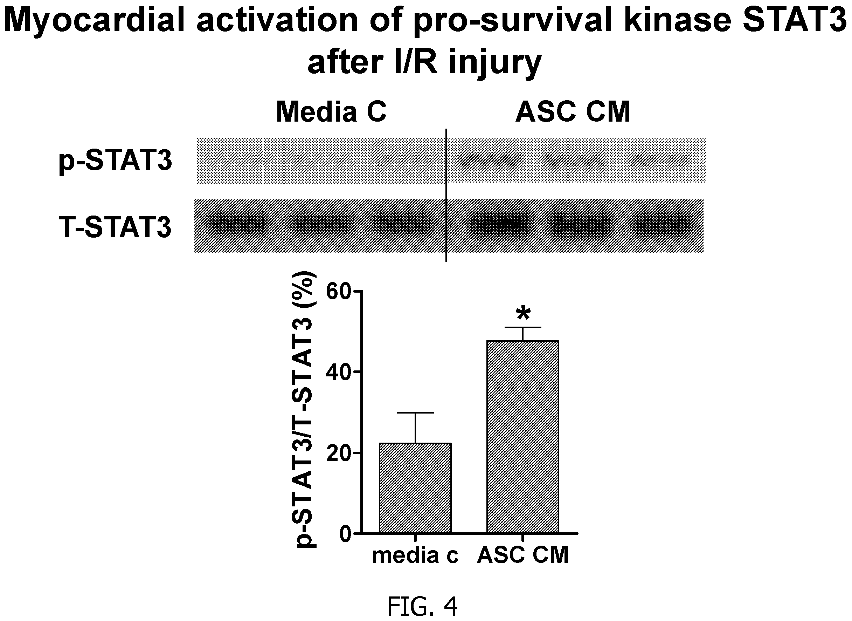

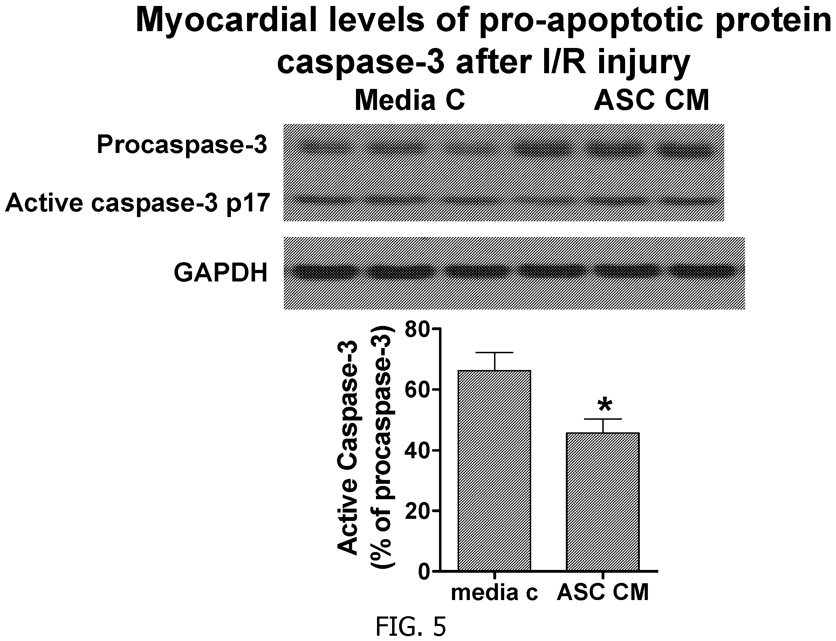

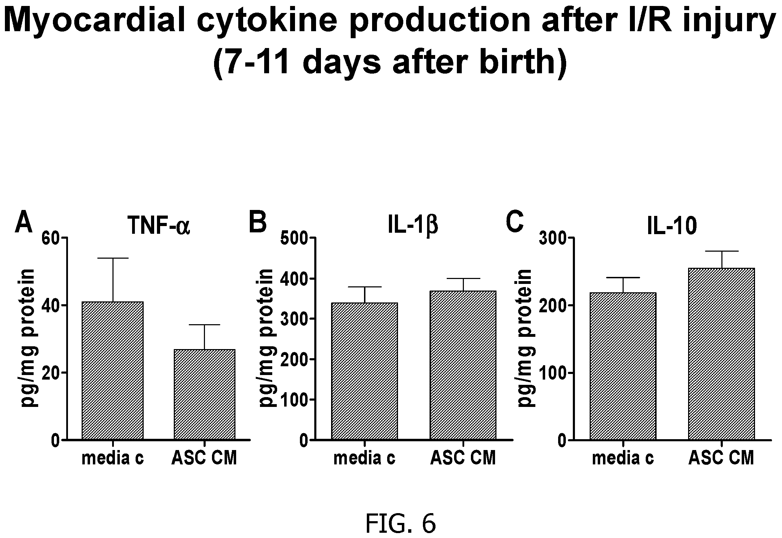

Referring now to FIGS. 4 and 5, ASC-CM-increased functional recovery was associated with up-regulated myocardial STAT3 activation and reduced apoptotic protein caspase-3 levels. However, pre-treatment with ASC-CM did not induce any significant changes to the production of myocardial cytokines (FIG. 6). These results suggest use of ASC-based therapy may be suitable for pediatric patients having undergone cardiac operations.

### Treatment of cardiac tissue with MSC-CM post-ischemia

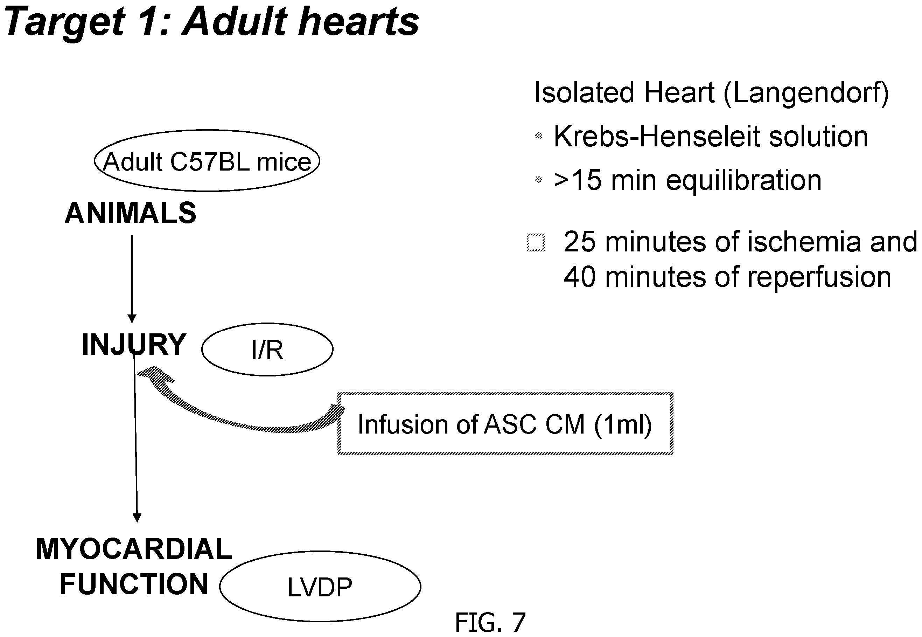

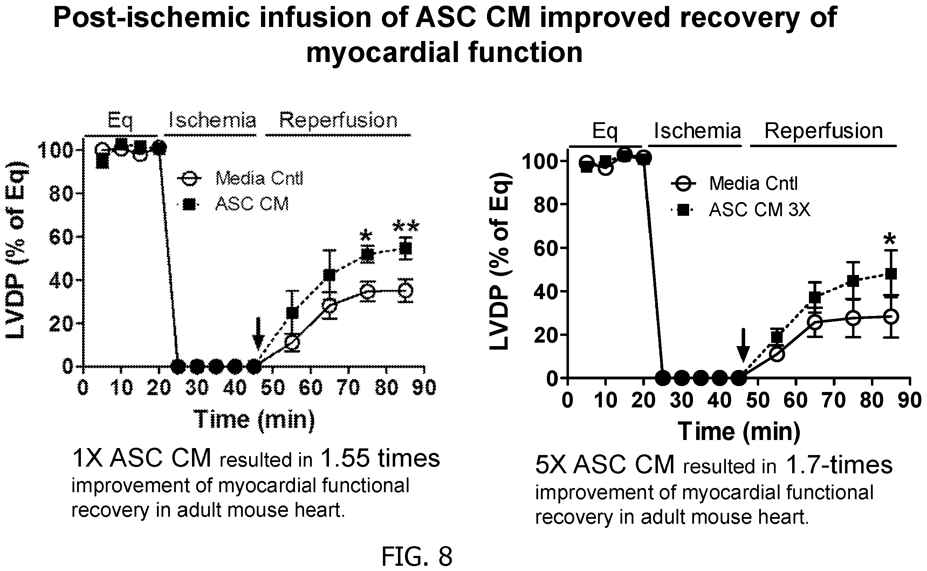

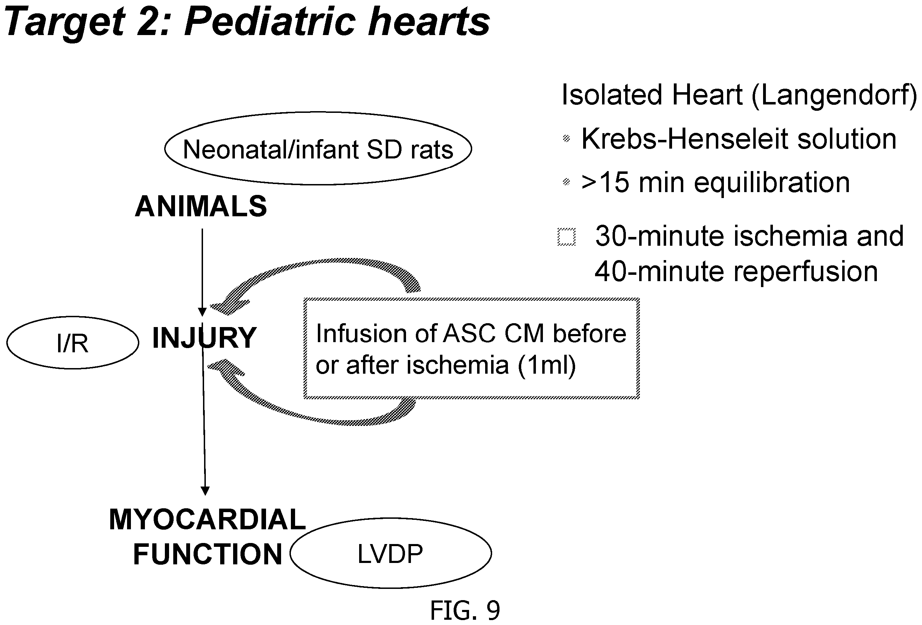

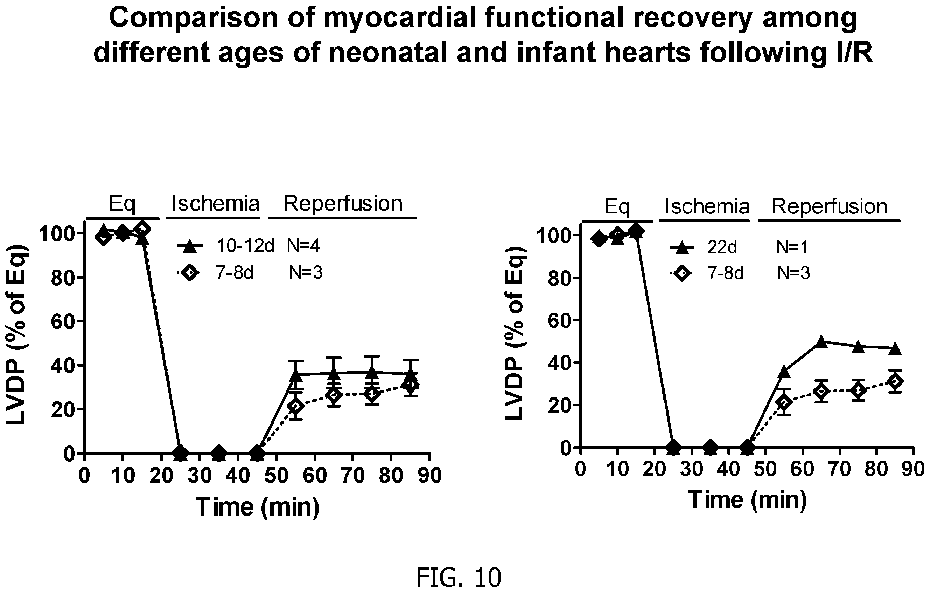

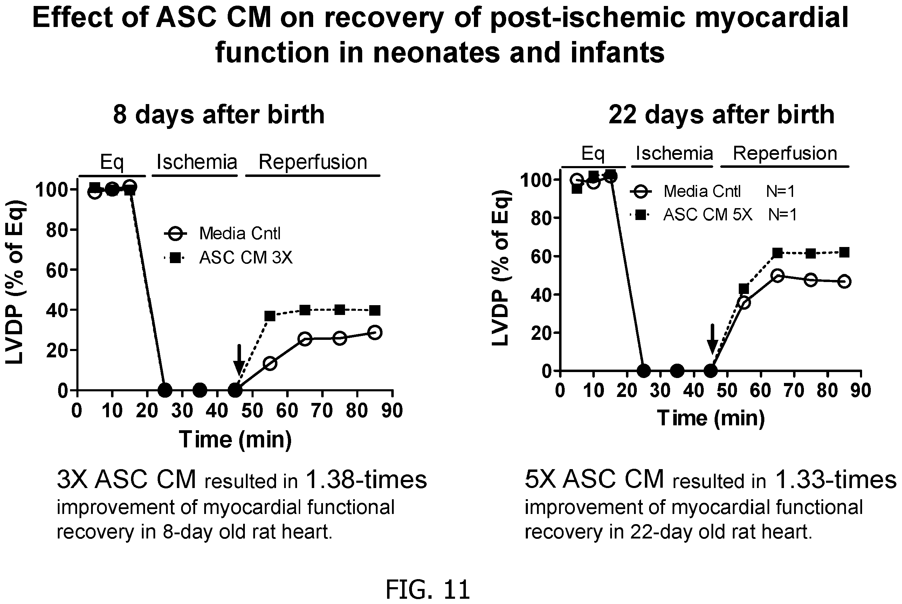

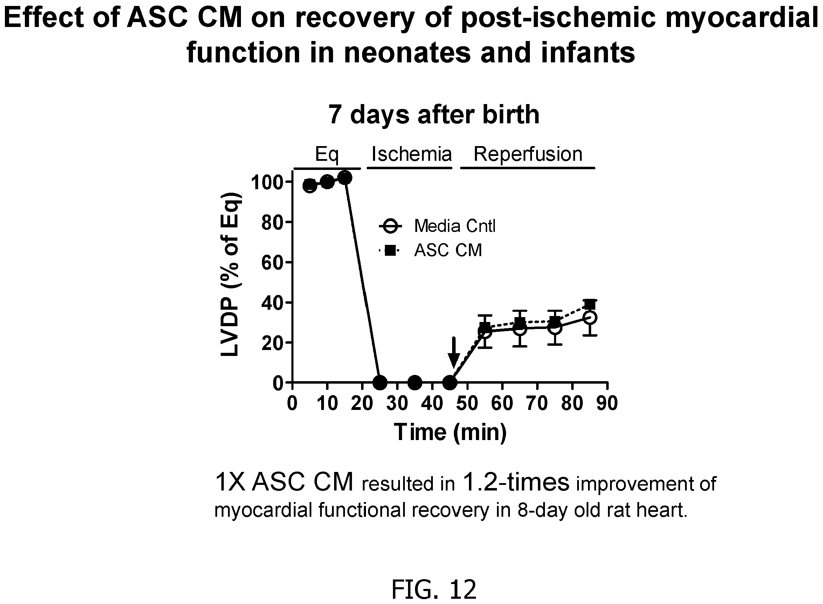

Referring now to FIGS 7-12, ASC-CM was delivered into the myocardium isolated adult mouse hearts, neonatal and infant rat hearts, after a global ischemic event. Post-ischemic infusion of ASC-CM significantly improved myocardial functional recovery in adult mouse hearts in a dose-dependent manner (FIG. 8). Post-ischemic infusion of ASC-CM improved myocardial function in neonatal and infant mouse hearts in a dose-dependent manner (FIGS. 10-12).

### Example 2: Transcriptomic profiling of cold-preserved cardiac tissue exposed to 6 hours of ischemia

### Methods

Isolated mouse hearts (4 hearts/treatment) were treated under following four conditions, mimicking those used to transport human hearts and/or the treatment provided herein:
1. Group I: normal
2. Group II: 6 hours of ischemia
3. Group III: 6 hours of ischemia with ASC factors
4. Group IV: 6 hours of ischemia with ASC factors collected under low oxygen

20,000,000 RNAs were sequenced from each heart using known methods. ∼ 13,000 genes were identified and quantitated (1-600,000) using known methods.

### Transcriptomic profiling of cardiac tissue before and during ischemia.

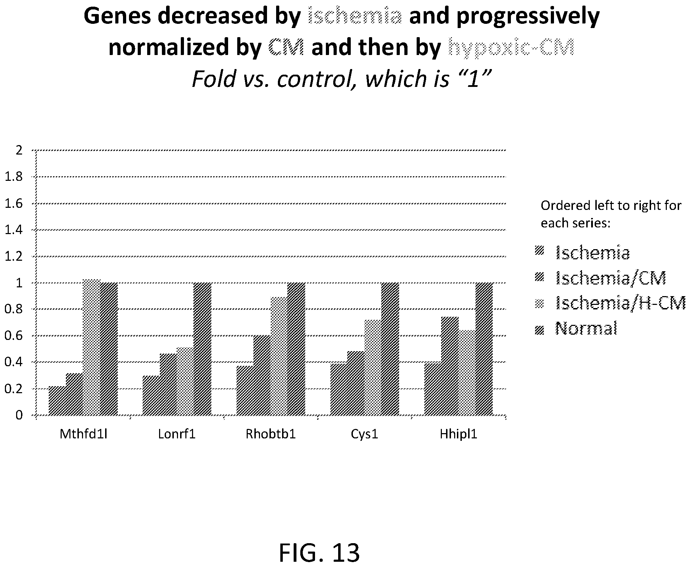

Referring now to FIG. 13, transcript expression of Mthfdl , Lonrf1, Rhobtbl , Cys 1, HhipM and Fam 107a were decreased during ischemia (relative to normal) and progressively restored towards their normal non-ischemic levels ("1 ") by treatment with Ad-MSC CM and Ad-MSC-CM obtained during hypoxia (H-CM) .

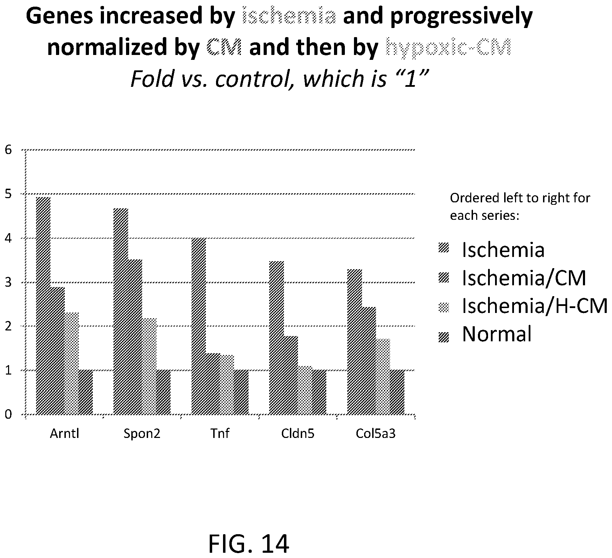

Referring now to FIG. 14, transcript expression of Arntl, Spon2, Tnf, Cldn5, Col5a3 and Sic41 a3 were increased during ischemia (relative to normal) and progressively restored towards their normal non-ischemic levels ("1 ") by treatment with Ad-MSC CM and Ad-MSC-CM obtained during hypoxia (H-CM).

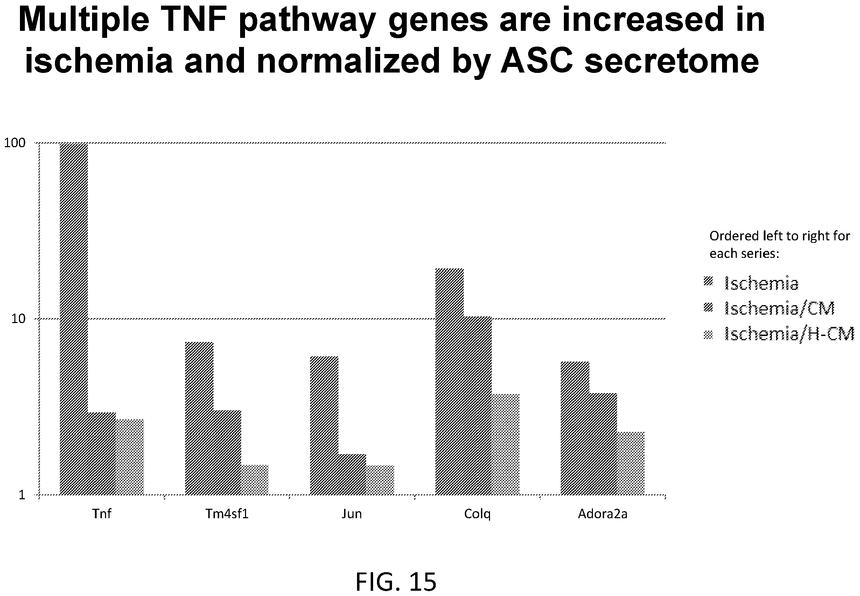

Referring now to FIG. 15, transcript expression of multiple TNF pathway genes are increased during ischemia (blue) progressively restored towards their normal non-ischemic levels ("1 ") upon treatment with Ad-MSC-CM and Ad-MSC-H-CM obtained during hypoxia.

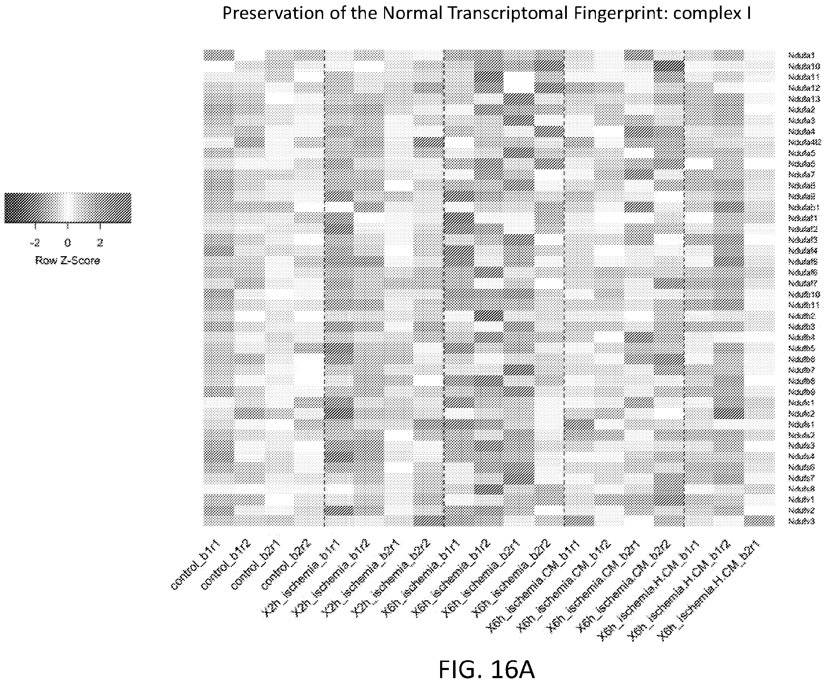

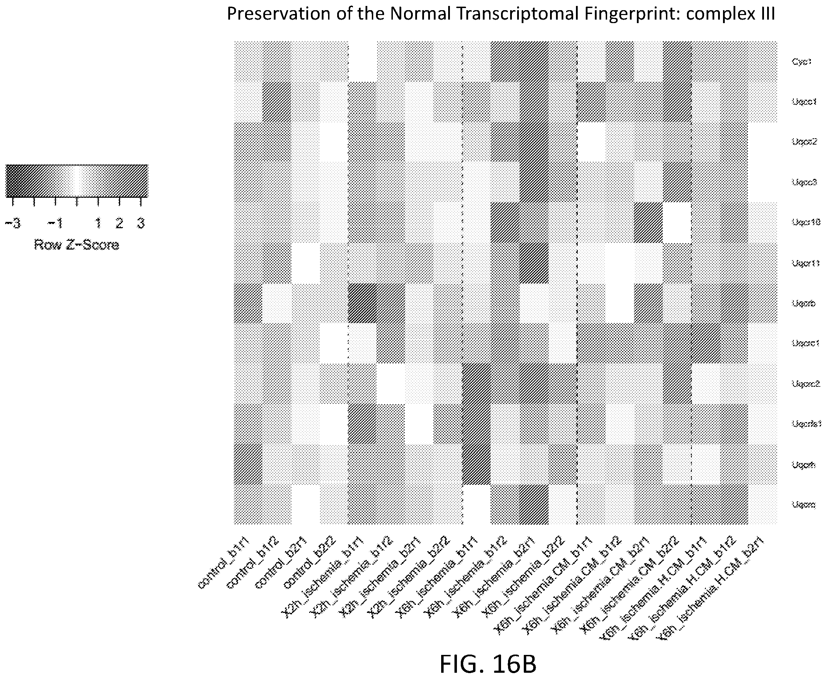

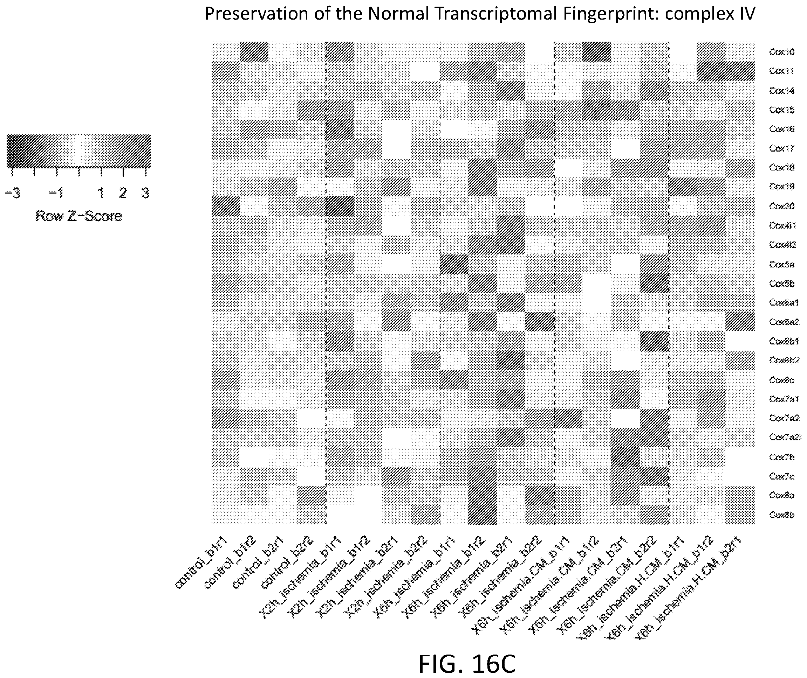

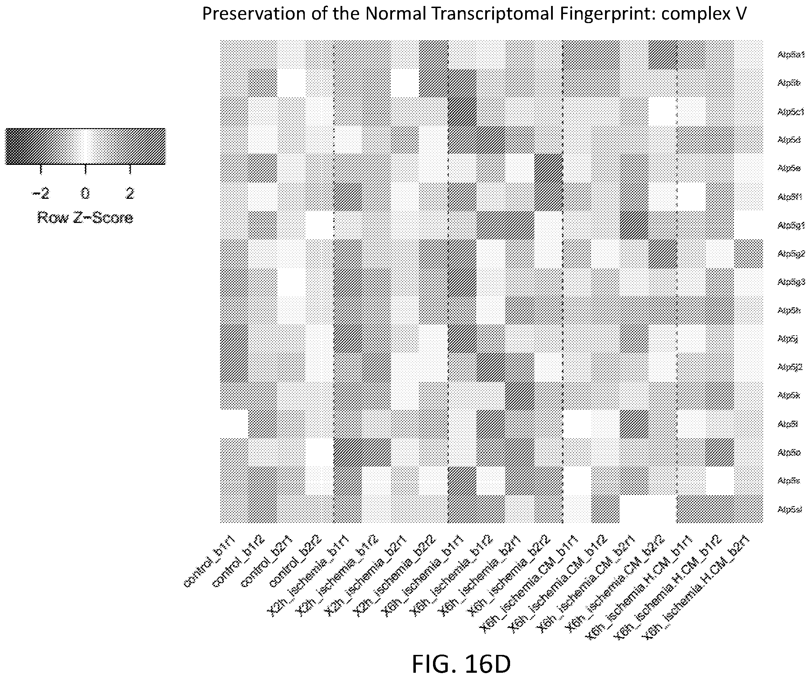

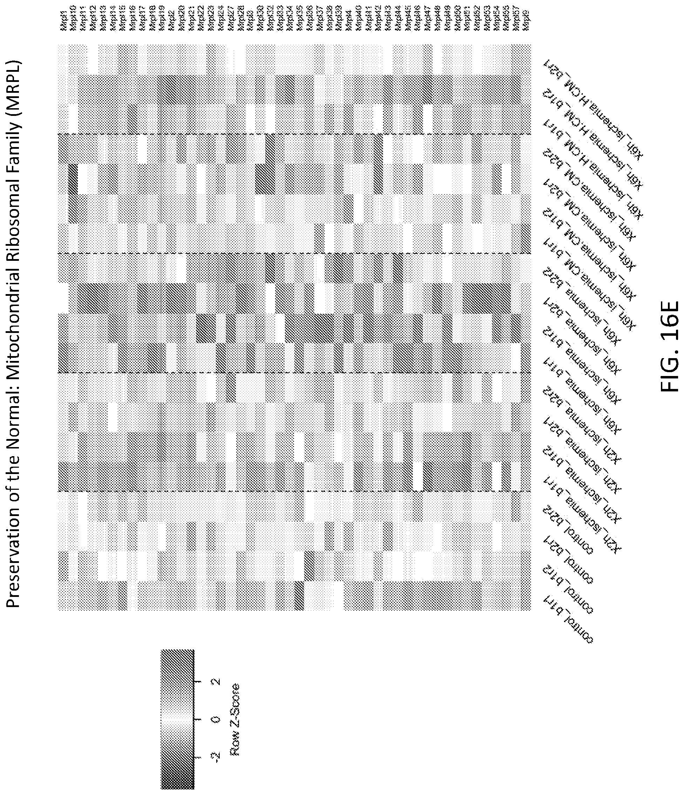

Referring now to FIGS. 16A-E, transcript expression of multiple mitochondrial electron transport chain complexes (I, III, IV and V) and the mitochondrial ribosomal family were differentially expressed in ischemic tissue and progressively restored towards their normal non-ischemic levels upon treatment with Ad-MSC-CM

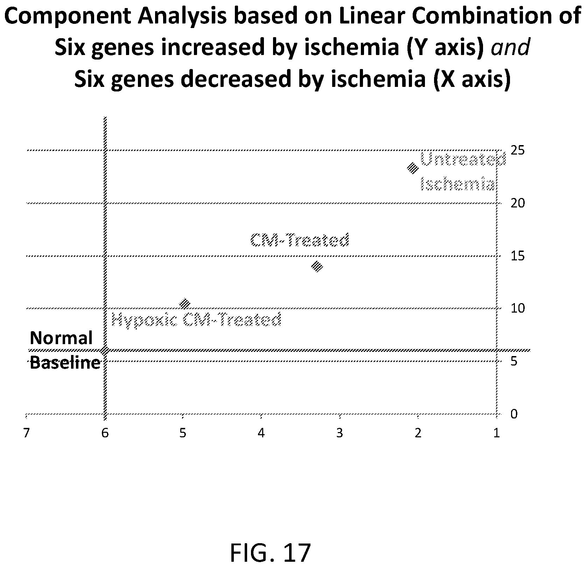

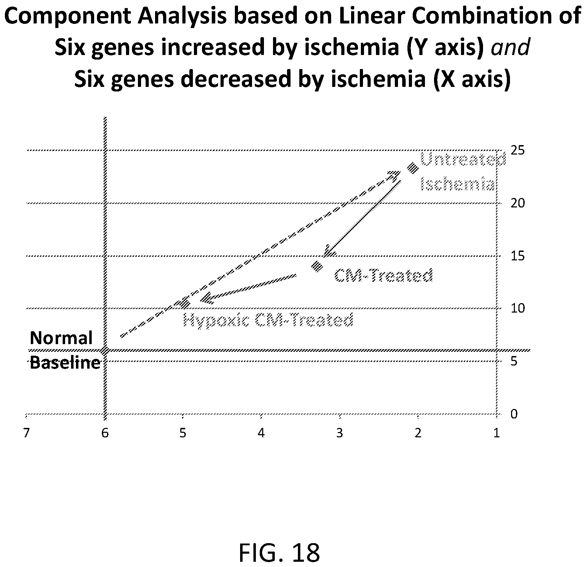

Referring now to FIGS. 17 and 18, component analysis based on linear combination of six genes increased by ischemia (Y axis) and six genes decreased by ischemia (X axis) , confirms progressive restoration of transcript expression towards normal by treatment with Ad-MSC-CM and Ad-MSC-H-CM.

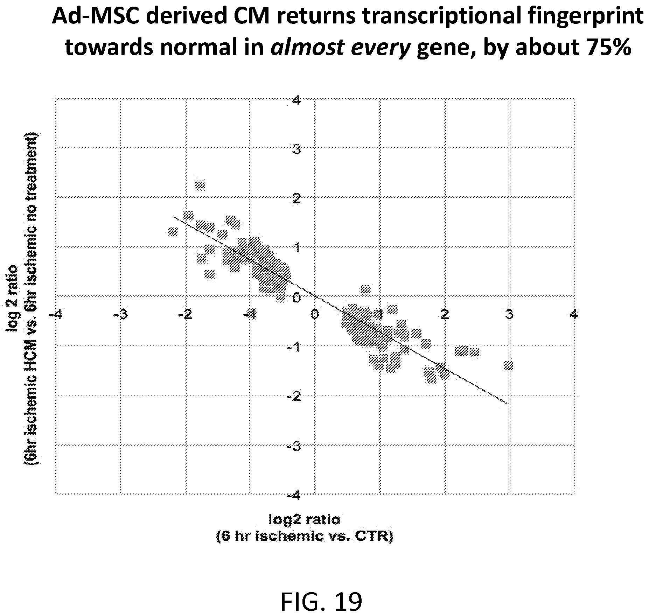

Referring now to FIG. 19, Ad-MSC-H-CM potently restores gene expression towards non-ischemic control levels. The log₂ ratio of expression in untreated ischemic hearts/normal non-ischemic hearts is plotted on the X-axis, and the log₂ ratio of expression in ischemic hearts exposed to hypoxic Ad-MSC CM (HCM) /untreated ischemic hearts. The transcriptional "fingerprint" demonstrates widespread restoration towards normal, reflected by the negative (-0.73) slope of the correlation line. The correlation coefficient is =0.94. FIG. 19 shows the relationship between the perturbation of gene expression in ischemia compared with control (CTR), on the X-axis, and the protection of gene expression during ischemia by exposure to hypoxic Ad-MSC CM (HCM) compared with ischemia alone, on the Y-axis. Importantly, for almost all genes, the degree and direction of change in ischemia vs. control is nearly reversed by the exposure to HCM. The correlation shows that on the average, in treated hearts, each gene is about 75% "restored" towards its normal non¬ ischemic value; genes increased by ischemia are decreased by treatment, while genes decreased by ischemia are increased by treatment.

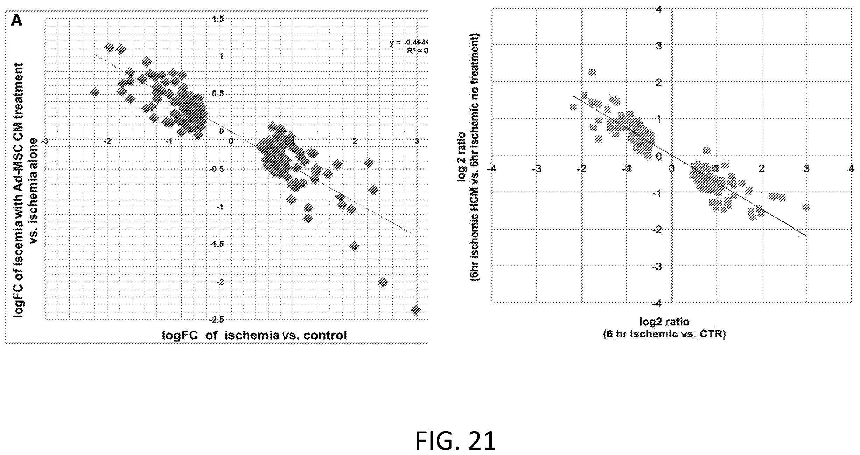

Referring now to FIGS. 20 and 21, Ad-MSC-CM treatment returns cardiac tissue transcriptional fingerprint towards normal in almost every gene, by about 46% , and Ad-MSC-H-CM treatment returns cardiac tissue transcriptional fingerprint towards normal in almost every gene, by about 75%.

### Example 3: Treatment of Cold-preserved Cardiac Tissue Exposed to 6 Hours of Ischemia

Human ASCs (hASCs) were cultured and expanded on tissue culture plates in EGM-2-MV medium and used for the experiments at passages 0 through 2. At 90% confluence, ASCs were switched to EBM-2/5% FBS. On the following day, the medium was replaced with fresh EBM-2/5% FBS, and ASCs were placed in either normoxic (21 % 0 ₂) or hypoxic (1% 0 ₂) conditions for 72 hours. ASCs can also be cultured in the presence of TNFD . At the end of the incubation period, the CM from ASCs was collected, and cell numbers were determined by a hemacytometer. Experiments were generally conducted in triplicate using media derived from each of the three donor cell cultures, to exclude idiosyncratic behavior of cells from a particular donor.

Mouse hearts were isolated, attached to the Langendorff apparatus, and immediately received coronary infusion of 1 ml of UW solution either without or with CM from Ad-MSC. The CM was concentrated by an eight-fold volume reduction via centrifugation through a 3 kD-cutoff molecular weight filter. The hearts were then placed in a tube containing 0.5 ml of the same solution and incubated in cold (4 °C), University of Wisconsin (UW) solution, emulating the conditions commonly used by medical centers during transport of donated hearts from explant to implantation. After 6 hours, the isolated hearts were assayed in the Langendorff system for their response to ischemia and reperfusion. Contractility values for untreated hearts were 40% of baseline, whereas CM-treated hearts demonstrated significantly improved function, maintaining 60% of the function of hearts not exposed to ischemia (FIG. 22).

Referring still to FIG. 22. Concentrated CM from Ad-MSC preserved function of isolated mouse hearts during conditions commonly used to transport human donor hearts. Mouse hearts were isolated from mice and immediately perfused on the Langendorff apparatus with 1 ml of UW solution either without or containing CM from Ad-MSC. (Final concentration of the CM was 8x original). The hearts (N=5 each group) were then placed in a tube containing 0.5 ml of the same solution and incubated at 4 °C for 6 hours. Finally, hearts were evaluated on the Langendorff system for their functional response to ischemia and reperfusion, except the hearts were allowed to contract spontaneously. Therefore the values are expressed as rate-pressure product (RPP) = spontaneous rate x left-ventricular developed pressure (LVDP).

The beneficial effects of the CM were confirmed by assaying the transcriptomes of the hearts from an experiment directly paralleling that above (FIG. 22), with RNA obtained from hearts (N=4 each group) following exposure to each of the following conditions: baseline (immediately frozen post-cardiectomy) ; 6 hours of cold ischemia after perfusion by UW solution; 6 hours of cold ischemia after perfusion by UW solution containing 8x CM from Ad-MSC cultured under standard conditions; and 6 hours of cold ischemia after perfusion by UW solution containing 8x CM from Ad-MSC cultured under hypoxic conditions (5% 0 ₂). Deep RNA sequencing was obtained for expression of 13,068 genes. In comparison with baseline (non-ischemic) control hearts, hearts exposed to 6 hours of ischemia demonstrated significant changes in expression in 184 genes (with a criterion of a false discovery rate (FDR)<0.05). However, in hearts treated with CM obtained from Ad-MSC incubated under standard conditions and then subjected to cold ischemia, only 14 genes showed significant changes in expression. Remarkably, hearts treated in parallel with CM from Ad-MSC incubated under hypoxia showed only 1 gene to be significantly altered from non-ischemic controls.

The 184 differentially expressed genes include: Lonrfl , Chd6, Rhobtbl , Wipf3, Raphl , Slc4 1a3, Per2, Colq, Cldn5, Timp3, Hlf, Per3, Bcl9, Apold I , Cys1 , Wee1 , Mthfd I ₗ, Col5a3, Sorbsl , Spon2, Slc43a3, Clmp, Rbp1 , Prickle3, Nfic, Slc6a6, Nxn, Gpcpd I , Tef, Podn, Mmp1 4, Smco4, SIc39a1 4, Eif5a2, Tm4sf1 , Slc4a8, Polr2a, Best3, Acotl , Xdh, Id 1, Usp2, Zbtb1 6, Sox4, Plcb4, Dusp1 8, 221 001 1C24Rik, Sex, Gja5, Plcd3, Arntl, Hspal b, Eepd I , Rcan I , Ppp1 r3a, Palld, Mylk4, CobiM , Nppb, Plekho2, Sox7, Cry2, Tmem 171, Vamp5, Dpy1 9l3, Dnajbl , Mrpl28, Fry, Flt1 , Neurl3, Naca, Neb, Bmp4, Hif3a, Npc1, Phf5a, Ccrn4l, Lrrc52, Synpo2, Cntfr, Ppfia4, Inhbb, Acot1 1, Sh2d4a, Ciart, Dctppl , Cipc, Naa60, Leo1 , Rgs1 6, Sik3, Gm1541 7, Pik3r1 , Gem, Slco5a1 , Gng 11, Wnk2, Fam 107a, Arhgap20, Guk1 , Mapkl o, Herpud I , Nme3, Zmizl , Ubap2, Fosl2, HyaM , Gbp5, Pdcd7, Jun, HhipM , Mcf2l, Cox6a1 , Ptprm, Dvl3, Fam2 12a, Adh 1, Smim20, Vwa1 , Tmtc1 , Hspal ₐ, Fxn, Fkbp2, Eda, Cdpfl , Cdc42bpa, ligp1 , Sorbs2, Lztsl , Clic5, Ctnnbipl , Actn I , Fmo2, Mid i ip1 , Paqr6, Tmem37, Atf7ip, Fis1 , Foxo3, Adamtsl4, S 100a1 6, Tnf, Ncoa3, Sp2, Gas1 , Vstm4, Unci 19b, Cry1 , Ptpn 18, Lmo4, Rash 1a, Pcdh I , Irs1 , Myeov2, Adora2a, Rrebl , Phf1 9, Rem1 , Man2a1 , Atpl Od, Vamp8, Ttpal, Ucp2, Sertad I , Usp54, Ncor2, ler2, Dnal4, Bri3bp, Mbnl2, Prepl, Uqcr1 1, 221 0407C1 8Rik, Epasl , Gngt2, Thra, Ptk2b, Hint2, Ubr2, Plcg2, Gimapl , Stk35, Ndufb9 and Wnt5b.

These data demonstrate that administration of Ad-MSC conditioned medium during ischemic storage confers significant preservation of function upon reperfusion, which is accompanied by marked preservation of the normal transcriptional "fingerprint" despite the ischemic period; and that the conditioned medium collected from Ad-MSC pre-exposed to hypoxia is more effective at preserving the normal transcriptional state.

## Claims

1. A composition for use in the prevention or treatment of ischemic injury and/or reperfusion injury and/or injury to the heart comprising:
- at least a portion of a mesenchymal stem cell conditioned medium (MSC-CM), wherein the MSC-CM comprises medium conditioned by contact with MSCs derived from adipose tissue; and
- the MSCs have been treated under hypoxic conditions selected from less than 10% O₂, less than 5% O₂, or 1% O₂; and wherein the MSCs have been treated with TNF-alpha.

2. The composition for use according to claim 1, wherein the at least a portion of MSC-CM comprises exosomes separated from the MSC-CM.

3. An *ex vivo* method of treating tissue comprising:
- contacting the tissue with a composition comprising at least a portion of MSC-conditioned medium (MSC-CM) for a treatment period, wherein the MSC-CM comprises medium conditioned by contact with MSCs derived from adipose tissue;
the MSCs have been treated under hypoxic conditions selected from less than 10% O₂, less than 5% O₂, or 1% O₂; and
wherein the MSCs have been treated with TNF-alpha;
wherein the tissue is cardiac tissue, and the tissue is comprised by an organ, wherein the organ is stored *ex vivo* or *ex-corporeal.*

4. The method of claim 3, wherein the tissue is cardiac tissue comprised by a heart, and wherein the heart is an adult heart, an infant heart, or a neonatal heart.

5. The method of any one of claims 3 or 4, wherein the contacting step comprises perfusing the organ tissue with the composition for the treatment period; and/or wherein the contacting step prevents, at least in part, ischemic damage in the tissue, thereby improving and/or preserving tissue function; and/or wherein the contacting step prevents, at least in part, reperfusion damage in the tissue, thereby improving and/or preserving tissue function.

6. The method of any one of claims 3-5, further comprising:
- analyzing the transcriptome of the contacted tissue; and
- comparing the transcriptome of the contacted tissue to a baseline transcriptome measured in a matched non-ischemic tissue, thereby evaluating the tissue;
wherein the analyzing step comprises analysis of one or more of following genes: Lonrf1, Chd6, Rhobtb1, Wipf3, Raph1, Slc41a3, Per2, Colq, Cldn5, Timp3, Hlf, Per3, Bcl9, Apold1, Cys1, Wee1, Mthfd1l, Col5a3, Sorbs1, Spon2, Slc43a3, Clmp, Rbp1, Prickle3, Nfic, Slc6a6, Nxn, Gpcpd1, Tef, Podn, Mmp14, Smco4, Slc39a14, Eif5a2, Tm4sf1, Slc4a8, Polr2a, Best3, Acot1, Xdh, Id1, Usp2, Zbtb16, Sox4, Plcb4, Dusp18, 2210011C24Rik, Sex, Gja5, Plcd3, Arntl, Hspa1b, Eepd1, Rcan1, Ppp1r3a, Palld, Mylk4, Cobll1, Nppb, Plekho2, Sox7, Cry2, Tmem171, Vamp5, Dpy19l3, Dnajb1, Mrpl28, Fry, Flt1, Neurl3, Naca, Neb, Bmp4, Hif3a, Npc1, Phf5a, Ccrn4l, Lrrc52, Synpo2, Cntfr, Ppfia4, Inhbb, Acot11, Sh2d4a, Ciart, Dctpp1, Cipc, Naa60, Leo1, Rgs16, Sik3, Gm15417, Pik3r1, Gem, Slco5a1, Gng11, Wnk2, Fam107a, Arhgap20, Guk1, Mapk10, Herpud1, Nme3, Zmiz1, Ubap2, Fosl2, Hyal1, Gbp5, Pdcd7, Jun, Hhipl1, Mcf2l, Cox6a1, Ptprm, Dvl3, Fam212a, Adh1, Smim20, Vwa1, Tmtc1, Hspa1a, Fxn, Fkbp2, Eda, Cdpf1, Cdc42bpa, ligp1, Sorbs2, Lzts1, Clic5, Ctnnbip1, Actn1, Fmo2, Midiip1, Paqr6, Tmem37, Atf7ip, Fis1, Foxo3, Adamtsl4, S100a16, Tnf, Ncoa3, Sp2, Gas1, Vstm4, Unc119b, Cry1, Ptpn18, Lmo4, Rasl11a, Pcdh1, Irs1, Myeov2, Adora2a, Rreb1, Phf19, Rem1, Man2a1, Atp10d, Vamp8, Ttpal, Ucp2, Sertad1, Usp54, Ncor2, ler2, Dnal4, Bri3bp, Mbnl2, Prepl, Uqcr11, 2210407C18Rik, Epas1, Gngt2, Thra, Ptk2b, Hint2, Ubr2, Plcg2, Gimap1, Stk35, Ndufb9 and Wnt5b;
wherein the tissue is evaluated as suitable for transplantation if expression of one or more of ARNT1, TNF, CLDN5, Col5a3, and SIc41a3 is the same or decreased relative to the baseline transcriptome and/or if expression of one or more of MTHFD1, LONRF1, RHOBTB1, Cycs1, Hhipl1, and FAM107a is the same or increased relative to the baseline transcriptome; or the tissue is evaluated as unsuitable for transplantation if expression of one or more of ARNT1, TNF, CLDN5, Col5a3, and SIc41a3 is increased relative to the standard and/or if expression of one or more of MTHFD1, LONRF1, RHOBTB1, Cycs1, Hhipl1, and FAM107a is decreased relative to the baseline transcriptome.

7. The method of claim 6, wherein the transcriptomic profile of the tissue is determined before the contacting step, during the contacting step, and/or after the contacting step; and wherein the results of the comparison are used to determine further treatment of the tissue with the composition; and/or wherein the results of the comparison are used to identify suitability of the tissue for transplant.

8. The method of any one of claims 3-7, further comprising contacting the tissue with a composition comprising at least a portion of MSC-CM for a treatment period, and repeating comparison of the transcriptome of the contacted tissue to a baseline transcriptome measured in a matched set of tissue, wherein the tissue is evaluated as suitable for transplantation if, according to the repeated comparison, expression of one or more of ARNT1, TNF, CLDN5, Col5a3, and SIc41a3 is the same or decreased relative to the baseline transcriptome and/or if expression of one or more of MTHFD1, LONRF1, RHOBTB1, Cycs1, Hhipl1, and FAM107a is the same or increased relative to the baseline transcriptome.

## Patentansprüche

1. Zusammensetzung zur Verwendung bei der Vorbeugung oder Behandlung von ischämischer Beschädigung und/oder Reperfusionsbeschädigung und/oder Beschädigung des Herzens, umfassend:
- mindestens einen Teil eines mesenchymalen Stammzell-konditionierten Mediums (MSC-CM), wobei das MSC-CM ein Medium umfasst, das durch Kontakt mit aus Fettgewebe gewonnenen MSCs konditioniert wird; und
- die MSCs unter hypoxischen Bedingungen behandelt wurden, die ausgewählt sind aus weniger als 10 % O₂, weniger als 5 % O₂ oder 1 % O₂; und wobei die MSCs mit TNF-alpha behandelt wurden.

2. Zusammensetzung zur Verwendung gemäß Anspruch 1, wobei der mindestens eine Teil von MSC-CM von dem MSC-CM getrennte Exosome umfasst.

3. Ex-vivo-Verfahren zur Behandlung von Gewebe, umfassend:
- Kontaktieren des Gewebes mit einer Zusammensetzung, die mindestens einen Teil eines MSC-konditionierten Mediums (MSC-CM) umfasst, für einen Behandlungszeitraum, wobei das MSC-CM ein Medium umfasst, das durch Kontakt mit aus Fettgewebe gewonnenen MSCs konditioniert wird;
die MSCs unter hypoxischen Bedingungen behandelt wurden, die ausgewählt sind aus weniger als 10 % O₂, weniger als 5 % O₂ oder 1 % O₂; und
wobei die MSCs mit TNF-alpha behandelt wurden,
wobei das Gewebe Herzgewebe ist und das Gewebe in einem Organ umfasst ist, wobei das Organ *ex vivo* oder *exkorporal* aufbewahrt wird.

4. Verfahren nach Anspruch 3, wobei das Gewebe Herzgewebe ist, das in einem Herz umfasst ist, und wobei das Herz ein Erwachsenenherz, ein Säuglingsherz oder ein Neugeborenenherz ist.

5. Verfahren nach einem der Ansprüche 3 oder 4, wobei der Kontaktierungsschritt das Perfundieren des Organgewebes mit der Zusammensetzung für den Behandlungszeitraum umfasst, und/oder wobei der Kontaktierungsschritt, mindestens teilweise, ischämischer Schädigung in dem Gewebe vorbeugt, wodurch die Gewebefunktion verbessert und/oder bewahrt wird; und/oder wobei der Kontaktierungsschritt, mindestens teilweise, Reperfusionsschädigung in dem Gewebe vorbeugt, wodurch die Gewebefunktion verbessert und/oder bewahrt wird.

6. Verfahren nach einem der Ansprüche 3-5, ferner umfassend:
- Analysieren des Transkriptoms des kontaktierten Gewebes; und
- Vergleichen des Transkriptoms des kontaktierten Gewebes mit einem Baseline-Transkriptom, das in einem übereinstimmenden nicht-ischämischen Gewebe gemessen wurde, wodurch das Gewebe beurteilt wird;
wobei der Analysierungsschritt die Analyse eines oder mehrerer der folgenden Gene umfasst: Lonrf1, Chd6, Rhobtb1, Wipf3, Raph1, Slc41a3, Per2, Colq, Cldn5, Timp3, Hlf, Per3, Bcl9, Apold1, Cys1, Wee1, Mthfd1l, Col5a3, Sorbs1, Spon2, Slc43a3, Clmp, Rbp1, Prickle3, Nfic, Slc6a6, Nxn, Gpcpd1, Tef, Podn, Mmp14, Smco4, Slc39a14, Eif5a2, Tm4sf1, Slc4a8, Polr2a, Best3, Acot1, Xdh, Id1, Usp2, Zbtb16, Sox4, Plcb4, Dusp18, 2210011C24Rik, Sex, Gja5, Plcd3, Arntl, Hspalb, Eepd1, Rcan1, Ppp1r3a, Palld, Mylk4, Cobll1, Nppb, Plekho2, Sox7, Cry2, Tmem171, Vamp5, Dpy19l3, Dnajb1, Mrpl28, Fry, Flt1, Neurl3, Naca, Neb, Bmp4, Hif3a, Npc1, Phf5a, Ccrn4l, Lrrc52, Synpo2, Cntfr, Ppfia4, Inhbb, Acot11, Sh2d4a, Ciart, Dctpp1, Cipc, Naa60, Leo1, Rgs16, Sik3, Gm15417, Pik3r1, Gern, Slco5a1, Gng11, Wnk2, Fam107a, Arhgap20, Guk1, Mapk10, Herpud1, Nme3, Zmiz1, Ubap2, Fosl2, Hyal1, Gbp5, Pdcd7, Jun, Hhipl1, Mcf2l, Cox6a1, Ptprm, Dvl3, Fam212a, Adh1, Smim20, Vwa1, Tmtc1, Hspa1a, Fxn, Fkbp2, Eda, Cdpf1, Cdc42bpa, ligp1, Sorbs2, Lzts1, Clic5, Ctnnbip1, Actn1, Fmo2, Mid1ip1, Paqr6, Tmem37, Atf7ip, Fis1, Foxo3, Adamtsl4, S100a16, Tnf, Ncoa3, Sp2, Gas1, Vstm4, Unc119b, Cry1, Ptpn18, Lmo4, Rasl11a, Pcdh1, Irs1, Myeov2, Adora2a, Rreb1, Phf19, Rem1, Man2a1, Atp10d, Vamp8, Ttpal, Ucp2, Sertad1, Usp54, Ncor2, Ier2, Dnal4, Bri3bp, Mbnl2, Prepl, Uqcr11, 2210407C18Rik, Epas1, Gngt2, Thra, Ptk2b, Hint2, Ubr2, Plcg2, Gimap1, Stk35, Ndufb9 und Wnt5b;
wobei das Gewebe als für eine Transplantation geeignet beurteilt wird, wenn die Expression eines oder mehrerer von ARNT1, TNF, CLDN5, Col5a3 und Slc41a3 relativ zu dem Baseline-Transkriptom gleich oder vermindert ist und/oder wenn die Expression eines oder mehrerer von MTHFD1, LONRF1, RHOBTB1, Cycs1, Hhipl1 und FAM107a relativ zu dem Baseline-Transkriptom gleich oder erhöht ist, oder das Gewebe als für eine Transplantation ungeeignet beurteilt wird, wenn die Expression eines oder mehrerer von ARNT1, TNF, CLDN5, Col5a3 und Slc41a3 relativ zu dem Standard erhöht ist und/oder wenn die Expression eines oder mehrerer von MTHFD1, LONRF1, RHOBTB1, Cycs1, Hhipl1 und FAM107a relativ zu dem Baseline-Transkriptom vermindert ist.

7. Verfahren nach Anspruch 6, wobei das transkriptomische Profil des Gewebes vor dem Kontaktierungsschritt, während des Kontaktierungsschritts und/oder nach dem Kontaktierungsschritt bestimmt wird; und wobei die Ergebnisse des Vergleichs verwendet werden, um weitere Behandlung des Gewebes mit der Zusammensetzung zu bestimmen; und/oder wobei die Ergebnisse des Vergleichs verwendet werden, um die Eignung des Gewebes für eine Transplantation zu identifizieren.

8. Verfahren nach einem der Ansprüche 3-7, ferner umfassend das Kontaktieren des Gewebes mit einer Zusammensetzung, die mindestens einen Teil von MSC-CM umfasst, für einen Behandlungszeitraum und Wiederholen des Vergleichs des Transkriptoms des kontaktierten Gewebes mit einem Baseline-Transkriptom, das in einem übereinstimmenden Gewebesatz gemessen wurde, wobei das Gewebe als für eine Transplantation geeignet beurteilt wird, wenn, gemäß dem wiederholten Vergleich, die Expression eines oder mehrerer von ARNT1, TNF, CLDN5, Col5a3 und Slc41a3 relativ zu dem Baseline-Transkriptom gleich oder vermindert ist und/oder wenn die Expression eines oder mehrerer von MTHFD1, LONRF1, RHOBTB 1, Cycs1, Hhipl1 und FAM107a relativ zu dem Baseline-Transkriptom gleich oder erhöht ist.

## Revendications

1. Composition destinée à être utilisée dans la prévention ou le traitement d'une lésion ischémique et/ou d'une lésion de reperfusion et/ou d'une lésion du coeur comprenant :
- au moins une partie d'un milieu conditionné par des cellules souches mésenchymateuses (MSC-CM), ledit MSC-CM comprenant un milieu conditionné par contact avec des MSC provenant du tissu adipeux ; et
- les MSC ont été traitées dans des conditions hypoxiques choisies parmi moins de 10 % d'O₂, moins de 5 % d'O₂ ou 1 % d'O₂ ; et lesdites MSC ayant été traitées avec du TNF-alpha.

2. Composition destinée à être utilisée selon la revendication 1, au moins une partie du MSC-CM comprenant des exosomes séparés du MSC-CM.

3. Méthode *ex vivo* de traitement d'un tissu comprenant :
- la mise en contact du tissu avec une composition comprenant au moins une partie de milieu conditionné par MSC (MSC-CM) pendant une période de traitement, ledit MSC-CM comprenant un milieu conditionné par contact avec des MSC provenant du tissu adipeux ;
lesdites MSC ayant été traitées dans des conditions hypoxiques choisies parmi moins de 10 % d'O₂, moins de 5 % d'O₂ ou 1 % d'O₂ ; et
lesdites MSC ayant été traitées avec du TNF-alpha ;
ledit tissu étant un tissu cardiaque, et ledit tissu étant constitué d'un organe, ledit organe étant stocké *ex vivo* ou *ex-corporeal.*

4. Méthode selon la revendication 3, ledit tissu étant un tissu cardiaque constitué d'un coeur, et ledit coeur étant un coeur d'adulte, un coeur de nourrisson ou un coeur de nouveau-né.

5. Méthode selon l'une quelconque des revendications 3 ou 4, ladite étape de mise en contact comprenant la perfusion du tissu organique avec la composition pendant la période de traitement ; et/ou ladite étape de mise en contact empêchant, au moins en partie, les lésions ischémiques dans le tissu, ce qui permet d'améliorer et/ou de préserver la fonction tissulaire ; et/ou ladite étape de mise en contact empêchant, au moins en partie, les lésions de reperfusion dans le tissu, ce qui permet d'améliorer et/ou de préserver la fonction tissulaire.

6. Méthode selon l'une quelconque des revendications 3 à 5, comprenant en outre :
- l'analyse du transcriptome du tissu mise en contact ; et
- la comparaison du transcriptome du tissu mis en contact à un transcriptome de base mesuré dans un tissu non ischémique apparié, ce qui permet d'évaluer le tissu ;
ladite étape d'analyse comprenant l'analyse d'un ou plusieurs des gènes suivants : Lonrf1, Chd6, Rhobtb1, Wipf3, Raph1, Slc41a3, Per2, Colq, Cldn5, Timp3, Hlf, Per3, Bcl9, Apold1, Cys1, Wee1, Mthfd1l, Col5a3, Sorbs1, Spon2, Slc43a3, Clmp, Rbp1, Prickle3, Nfic, Slc6a6, Nxn, Gpcpd1, Tef, Podn, Mmp14, Smco4, Slc39a14, Eif5a2, Tm4sf1, Slc4a8, Polr2a, Best3, Acot1, Xdh, Id1, Usp2, Zbtb16, Sox4, Plcb4, Dusp18, 2210011C24Rik, Scx, Gja5, Plcd3, Arntl, Hspalb, Eepd1, Rcan1, Ppplr3a, Palld, Mylk4, Cobll1, Nppb, Plekho2, Sox7, Cry2, Tmem171, Vamp5, Dpy19l3, Dnajb1, Mrpl28, Fry, Flt1, Neurl3, Naca, Neb, Bmp4, Hif3a, Npc1, Phf5a, Ccrn4l, Lrrc52, Synpo2, Cntfr, Ppfia4, Inhbb, Acot11, Sh2d4a, Ciart, Dctpp1, Cipc, Naa60, Leo1, Rgs16, Sik3, Gm15417, Pik3r1, Gem, Slco5a1, Gng11, Wnk2, Fam107a, Arhgap20, Guk1, Mapk10, Herpud1, Nme3, Zmiz1, Ubap2, Fosl2, Hyal1, Gbp5, Pdcd7, Jun, Hhipl1, Mcf21, Cox6a1, Ptprm, Dvl3, Fam212a, Adh1, Smim20, Vwa1, Tmtc1, Hspala, Fxn, Fkbp2, Eda, Cdpf1, Cdc42bpa, ligp1, Sorbs2, Lzts1, Clic5, Ctnnbip1, Actn1, Fmo2, Mid1ip1, Paqr6, Tmem37, Atf7ip, Fis1, Foxo3, Adamtsl4, S100a16, Tnf, Ncoa3, Sp2, Gas1, Vstm4, Unc119b, Cry1, Ptpn18, Lmo4, Rasl11a, Pcdh1, Irs1, Myeov2, Adora2a, Rreb1, Phf19, Rem1, Man2a1, Atp10d, Vamp8, Ttpal, Ucp2, Sertad1, Usp54, Ncor2, Ier2, Dnal4, Bri3bp, Mbnl2, Prepl, Uqcr11, 2210407C18Rik, Epas1, Gngt2, Thra, Ptk2b, Hint2, Ubr2, Plcg2, Gimap1, Stk35, Ndufb9 et Wnt5b ;
ledit tissu étant évalué comme étant approprié pour une transplantation si l'expression d'un ou plusieurs éléments parmi ARNT1, TNF, CLDN5, Col5a3 et Slc41a3 est identique ou réduite par rapport au transcriptome de base et/ou si l'expression d'un ou plusieurs éléments parmi MTHFD1, LONRF1, RHOBTB1, Cycs1, Hhipl1 et FAM107 est identiques ou accrue par rapport au transcriptome de base ; ou ledit tissu étant évalué comme impropre à une transplantation si l'expression d'un ou plusieurs éléments parmi ARNT1, TNF, CLDN5, Col5a3 et Slc41a3 est accrue par rapport à la norme et/ou si l'expression d'un ou plusieurs éléments parmi MTHFD1, LONRF1, RHOBTB1, Cycs1, Hhipl1 et FAM107a est réduite par rapport au transcriptome de base.

7. Procédé selon la revendication 6, le profil transcriptomique du tissu étant déterminé avant l'étape de mise en contact, pendant l'étape de mise en contact et/ou après l'étape de mise en contact ; et lesdits résultats de la comparaison étant utilisés pour déterminer un traitement ultérieur du tissu avec la composition ; et/ou lesdits résultats de la comparaison étant utilisés pour identifier l'aptitude du tissu à être transplanté.

8. Procédé selon l'une quelconque des revendications 3 à 7, comprenant en outre la mise en contact du tissu avec une composition comprenant au moins une partie du MSC-CM pendant une période de traitement, et la comparaison répétée du transcriptome du tissu mis en contact avec un transcriptome de base mesuré dans un ensemble de tissu apparié, ledit tissu étant évalué comme étant approprié pour une transplantation si, selon la comparaison répétée, l'expression d'un ou plusieurs parmi ARNT1, TNF, CLDN5, Col5a3 et Slc41a3 est identique ou réduite par rapport au transcriptome de base et/ ou si l'expression d'un ou plusieurs éléments parmi MTHFD1, LONRF1, RHOBTB1, Cycs1, Hhipl1 et FAM107a est identique ou accrue par rapport au transcriptome de base.
